# EUROPEAN PATENT SPECIFICATION

(11) **EP 2 362 942 B1**
(45) Date of publication and mention of the grant of the patent: **21.06.2017**
(21) Application number: 09752320.3
(22) Date of filing: 30.10.2009
(51) Int. Cl.: G01N 33/68, G06F 19/00

(54) **BIOMARKERS**
BIOMARKER
BIOMARQUEURS

(30) Priority: 30.10.2008 US 109649 P
(43) Date of publication of application: 07.09.2011
(73) Proprietor: Luxembourg Institute of Health, 1445 Strassen (LU)
(72) Inventor: DEVAUX, Yvan, F-57330 Zoufftgen (FR); WAGNER, Daniel R., L-8083 Bertrange (LU); AZUAJE, Francisco, L-8010 Strassen (LU); VAUSORT, Mélanie, L-8041 Strassen (LU)
(74) Representative: Paemen, Liesbet R.J.
(86) International application number: PCT/EP2009/064410
(87) International publication number: WO 2010/049538

(56) References cited:
- WO-A1-01/88181
- WO-A2-03/020120
- SILVESTRE JEAN-SÉBASTIEN ET AL: "Vascular endothelial growth factor-B promotes in vivo angiogenesis." CIRCULATION RESEARCH 25 JUL 2003, vol. 93, no. 2, 25 July 2003 (2003-07-25), pages 114-123, XP002564893 ISSN: 1524-4571
- BELLOMO D ET AL: "Mice lacking the vascular endothelial growth factor-B gene (Vegfb) have smaller hearts, dysfunctional coronary vasculature, and impaired recovery from cardiac ischemia." CIRCULATION RESEARCH 4 FEB 2000, vol. 86, no. 2, 4 February 2000 (2000-02-04), pages E29-E35, XP002564894 ISSN: 1524-4571

## Description

### FIELD OF THE INVENTION

The present invention relates to a set of new biomarkers for assessing the risk of Heart Failure (HF) in a patient, particularly after the patient has suffered from a myocardial infarction (MI). Further described are diagnostic kits to measure levels of these biomarkers, the kits not being part of the invention.

### BACKGROUND OF THE INVENTION

Heart failure (HF) is not a specific disease, but a compilation of signs and symptoms, all of which are caused by an inability of the heart to appropriately increase cardiac output as needed. Patients typically present with shortness of breath, edema and fatigue. HF has become a disease of epidemic proportion, affecting 3 % of the adult population. Mortality of HF is worse than many forms of cancer with a five-year survival of less than 30 %. Myocardial infarction (MI) is one of the leading causes of HF. 63% of the patients develop HF in the 6 years following MI. Left ventricular remodeling contributes largely to HF. Because HF becomes more common in the elderly, the number of affected individuals will continue to rise with our ageing population. Extensive research in the last decade has brought a better understanding of the pathophysiology of HF. At the same time, many new therapeutic targets have been identified, although only a few of them are of potential therapeutic use. Some of these targets have been called "prognostic biomarkers" or "biomarkers" when their prognostic value could be demonstrated.

Biomarkers can be classified into three categories. Biomarkers that can assist in the care of apparently healthy individuals are called "screening biomarkers." Biomarkers seen in patients having a suspicion of disease are called "diagnostic biomarkers," and biomarkers seen in patients with overt disease are called "prognostic biomarkers." While diagnostic biomarkers such as troponin I and troponin T for MI and brain natriuretic peptide (BNP) for heart failure are used in clinical practice, the potential use of these biomarkers as prognostic biomarkers to tailor the treatment to the individual patient ("personalized medicine") has yet to be proved.

It has to be noted that the earlier the identification of a patient prone to develop HF after MI can be made, the more efficiently the treatment can be adjusted. However, a major limitation of the use of individual biomarkers is that not all patients may present such risk factors independently. Therefore, it has become evident that the early identification of patients prone to develop HF after MI would considerably benefit from the multiplication and integration of biomarkers.

Vascular endothelial growth factor (VEGF), a sub-family of growth factors, is a broad term covering a number of proteins from a number of families. These growth factors have mostly been studied for their angiogenic properties. Various growth factors have been identified to date, the most well known of which is VEGFA. VEGFA is often simply referred to as "VEGF" (Ferrara et al. "The biology of VEGF and its receptors" Nat Med. 2003 jun;9(6):669-676). One of the proposed biomarkers of the present invention, VEGFB, is part of the family of vascular endothelial growth factors but is distinct from VEGFA.

### OBJECTS OF THE INVENTION

The objects of the present invention are:

### 1. To provide a prognostic tool

It is an object of the present invention to provide a tool for early prognosis of the occurrence of HF in order to improve survival and to lessen the development of worsening HF.

It is another object of the present invention to use this prognostic tool to identify patients at risk to develop heart failure.

It is another object not being part of the invention to use this prognostic tool to adjust treatments to better prevent the development of ventricular remodeling and HF after MI.

### 2. To provide a new diagnostic kit

It is an object, not being part of the invention, to provide a diagnostic kit able to measure marker concentrations in biological fluids in order to help in the early prognosis of the occurrence of HF to improve survival and to lessen the development of worsening HF.

It is another object, not being part of the invention, to provide a new diagnostic kit to identify patients at risk to develop ventricular remodeling and HF.

It is another object, not being part of the invention, to provide a new diagnostic kit to adjust treatments to better prevent the development of ventricular remodeling and HF after MI. Furthermore, it is also an object of the present invention to provide biomarkers that can be used in screening patients, post-MI, for the susceptibility of a patient to develop HF or Ventricular Remodeling.

Surprisingly, we have found that the mRNA and plasma levels of the following proteins VEGFB, THBS1 and PGF vary post MI and are excellent indica of the likelihood of the patient to go on to develop HF and/or Ventricular Remodeling.

These biomarkers can, therefore, be used to screen MI patients and, in particular, provide an early prognostic tool for identifying those patients who, having suffered from MI, are at an increased risk of then going on to develop HF and/or Ventricular Remodeling. Diagnostic kits for measuring the levels of these three biomarkers are also provided and are useful in the context of MI to predict the occurrence of HF and/or Ventricular Remodeling.

### SUMMARY OF THE INVENTION

Disclosed herein is a method of identifying myocardially-infarcted patients having an increased risk of developing a heart condition, comprising:
- assaying, post infarction, a body fluid sample from the patient for levels of Vascular Endothelial Growth Factor B (VEGFB), Thrombospondin-1 (THBS1) and/or Placental Growth Factor (PGF);
- comparing the levels VEGFB, THBS1 and/or PGF with the corresponding levels of VEGFB, THBS1 and/or PGF from a reference sample, said reference sample having a known clinical outcome; and
- determining whether the patient has an increased risk of developing a heart condition, based on said comparison.

According to a first aspect, the present invention provides a method of identifying myocardially-infarcted patients having an increased risk of developing heart failure, comprising:
- assaying, post infarction, a body fluid sample from the patient for levels of Vascular Endothelial Growth Factor B (VEGFB), Thrombospondin-1 (THBS1) and/or Placental Growth Factor (PGF);
- comparing the levels of VEGFB, THBS1 and/or PGF with the corresponding levels of VEGFB, THBS1 and/or PGF with a control; and
- determining whether the patient has an increased risk of developing heart failure, based on said comparison.

In some embodiments, at least one of the following indicates an increased likelihood of said patient suffering from said heart condition:
- lower levels of VEGFB in the assayed patient sample compared to the VEGFB control level;
- higher levels of THBS1 in the assayed patient sample compared to the THBS1 control level; and/or
- higher levels of PGF in the assayed patient sample compared to the PGF control level.

As disclosed herein, the heart condition may be myocardial infarction, acute coronary syndrome, ischemic cardiomyopathy or non-ischemic cardiomyopathy. More preferably, the patient may go on to develop or suffer from heart failure. In some instances, the patient may undergo ventricular remodeling. It will be appreciated that many myocardially-infarcted patients undergo ventricular remodeling and subsequently, or at the same time, develop the condition known as heart failure. Therefore, there is a clear correlation between ventricular remodeling and heart failure and, preferably, myocardially infarcted patients undergoing ventricular remodeling will also develop heart failure.

As disclosed herein the body fluid sample taken from the patient may be a blood sample, a plasma sample or a serum sample. Preferably, the levels of VEGFB, THBS1 and PGF assayed are mRNA levels. These may be determined by assaying mRNA in red and/or white blood cells. Preferably, the blood cells are leukocytes, neutrophils, basophils, eosinophils, lymphocytes, monocytes, platelets, or erythrocytes.

Preferably, the VEGFB, THBS1 and PGF may be measured at the mRNA level from blood cells by any technique able to quantitate mRNA, most preferably quantitative PCR, most preferably microarrays. It is also preferred that, the VEGFB, THBS1 and PGF may be measured at the protein level in the plasma by any technique able to quantitate proteins, most preferably ELISA. mRNA assay results may be used in combination with plasma protein assay results for a more accurate assessment.

In some instances, not being part of the invention, only the level of PGF is assayed, which may preferably be any of the sequences for PGF given in SEQ ID NOS 7-9, or fragments thereof. In other instances, not being part of the invention, only the level of THBS1 is assayed, which may preferably be any of the sequences for THBS1 given in SEQ ID NOS 4-6, or fragments thereof. In other instances, not being part of the invention, both the levels of THBS1 and PGF are assayed.

In yet further instances, the level of VEGFB is assayed, which may preferably be any of the sequences for VEGFB given in SEQ ID NOS 1-3, or fragments thereof. This is in combination with THBS1 and PGF. It is preferred that the VEGFB may be either the splicing variant VEGFB 186, the splicing variant VEGFB 167 or both. As disclosed herein, the mRNA level may be assayed the day of myocardial infarction. mRNA or plasma protein samples for assaying may be obtained from the patient on day 1, being the day following myocardial infarction. A subsequent sample, obtained from the patient later on day 1 or on day 2 or 3 or 4 or 5 or 6 or 7 or at any time up to 1 month post infarction, may also be assayed.

As disclosed herein, the subsequent sample may be obtained from the patient on day 1 and the assayed value from day 1 is compared with the reference sample. However, in other instances, the subsequent sample is obtained from the patient on day 1 and the assayed value from day 1 is compared with the assayed levels in the sample from the day of infarction. This is most preferable in respect of VEGFB. In other words, the control, first post-MI and subsequent samples may be assayed for levels of VEGFB and these VEGFB levels are compared. An increase in VEGFB levels from the reference sample to day 1 or 2 is particularly useful in identifying the patient as being at lower risk of developing said condition. Similarly, a decrease in VEGFB levels from the control sample to day 1 or 2 is particularly useful in identifying the patient as being at a higher risk of developing said condition.

However, the VEGFB levels from the first post-MI sample at day 0 or 1 to the subsequent sample (post MI) at day 1 or 2 may be compared, as an increase in VEGFB from day 0 or 1 to day 1 or 2 is highly indicative of a patient with lowered risk of developing the present conditions. Changes in VEGFB measured between day 0 and day 2 or day 1 and day 3 are particularly preferred.

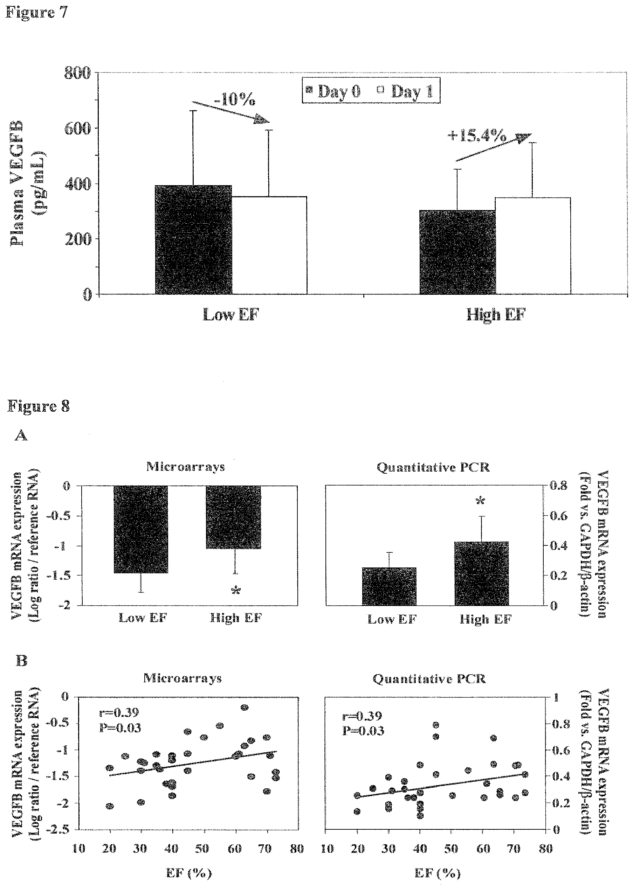

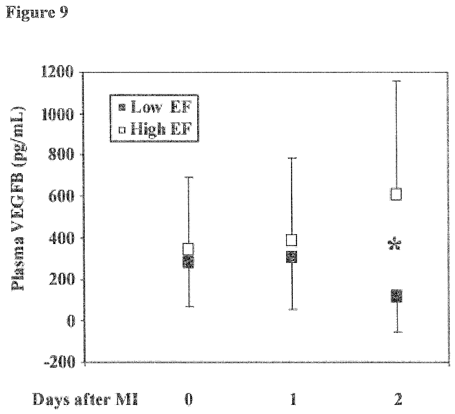

Indeed, we found that plasma VEGFB levels were similar between high and low EF groups at day 0 and day 1 after MI. At day 2 however, VEGFB levels increased in high EF patients (2 fold compared with day 0) whereas they dropped in low EF patients (2.5 fold compared with day 0) (*Figure 9*). These data are in accordance with the up
regulation of VEGFB mRNA in high EF patients (*Figure 8*). These results suggest that patients able to increase their production of VEGFB after MI are more prone to have a favourable outcome.

A decrease in VEGFB from day 0 or 1 to day 1 or 2, and in particular day 0 to 2, is highly indicative of a patient with an elevated risk of developing the present conditions.

The present invention measures changes in the levels of expression or prevalence of certain biomarkers, rather than just the presence or absence thereof, as is sometimes the case in the art. In order to measure a change in said levels, a point of reference needs to be established. This can preferably be the levels in a further sample, in particular an earlier sample, preferably taken on the day of infarction (day zero) or on the first or second day following infarction. Preferably, the control sample is the basal level of VEGFB, THBS1 or PGF on the day of infarction, respectively.

Alternatively, the control can be a reference value obtainable from a population of infracted patients with a known range of clinical outcomes. A database can be built up of data from infarcted patients and once calibrated for age, sex etc, an average (mode, mean or median as deemed appropriate) value or value range can be ascertained for patients having certain criteria (for instance sex, weight and age), measured at a particular time post infarction, and with known clinical outcomes (i.e. heart failure or not). The data from the assayed patient can then be compared against this reference value or range to determine the likelihood of the assayed patient having one or other of the clinical outcomes. For instance, if it established that VEGFB levels lower than around "X" micrograms\ml in the plasma of infarcted male patients aged 55-60 measured on day 1 post infarction gives a 50% likelihood of heart failure, then an assayed sample can be taken from a corresponding male on day 1 and compared to this value to determine if the 50% likelihood applies to this patient.

The determination step may be by suitable statistical analysis, for instance "nearest neighbor" comparison techniques, such as the Kstar and SVM programs. A particularly preferred example is to use a data mining platform, such as Weka. This may be followed by hierarchical clustering, preferably implemented using unweighted pair-group method with arithmetic averages and correlation coefficients. Clustering visualization may then be performed with GEPAS. Optionally, statistical significance tests, Pearson correlation values, and graphical plots may be generated with the Statistica package (v. 6.0).

The method may further comprise collecting data on one or more MI patients, the data preferably to include the levels or values of at least one of, and preferably each of, the three mRNAs and/or plasma proteins (VEGFB, THBS1 and/or PGF) and the associated clinical outcome for that patient. This is used to create feature/value data for VEGFB, THBS1 and/or PGF associated with a particular clinical outcome.

Thereafter, a database may be populated with the data from each individual. These known values could be referred to as reference (or "seen") samples.

"Query" or "unseen" sample data, i.e. from a recently infarcted new patient, is inputted and queried against the reference data in the database. The query data is obtained from a sample collected from a patient who has just had an MI and for whom it is desired to establish the likelihood of developing Heart Failure. In other words, the clinical outcome for this patient is unknown and the operator is asking the program to predict this patient's outcome (increased or reduced risk of HF post-MI).

The program compares the unseen/query sample data with the reference data set and makes its prediction on the clinical outcome of the patient.

Where reference is made to mRNA above, it will be appreciated that the same holds true for the plasma protein levels.

A classifier is preferred to determine a prognosis. The classifier may include programs such as PAM, Kstar and SVM which are well known to make a prediction in different ways. However, there is still always a comparison of the mRNA (or protein) data in the "unseen" sample with one or more of the "seen" data points, for instance when searching for the "nearest neighbor." Kstar and SVM, for instance, use different algorithms, but essentially work in similar ways by comparing the query data or values against the nearest reference set of values.

It is, therefore, preferred that the classifier searches the database and compares the query/unseen data with the known/seen data. Having found the "closest match" (for instance in a 3-D sense when analyzing all three mRNA or plasma protein levels) in the database, the program bases its prediction for the clinical outcome of the query patient based on the clinical outcome of that closet match.

Also provided is a method comprising:
- analyzing a body fluid sample from a post-MI patient for mRNA or plasma protein levels of VEGFB, THBS1 and PGF to determine feature data of the patient, associating this feature data with a particular clinical outcome relating to the incidence of Heart Failure in said patient, and entering this feature data into a database;
- repeating the analysis for a plurality of post-MI patients, to populate a database to contain reference information about a relationship between incidence of HF and levels of VEGFB, THBS1 and PGF;
- determining feature data of a post-MI patient with an unknown prognosis; and
- processing the feature data for the VEGFB, THBS1 and PGF levels from the patient to compare it with the feature data in the database; and
- outputting a likely prognosis for the post-MI unknown prognosis patient in dependence upon the result of the comparison.

These steps may also be included in the method according to the first aspect of the invention.

The likely prognosis may be an increased or reduced risk of HF, which can then affect the clinician's further proscribed treatment for the patient. The terms prognosis and diagnosis may be used interchangeably, unless otherwise apparent.

Also provided is a database comprising feature data from MI patients, the feature data including the clinical outcome of the patient matched to VEGFB, THBS1 and PGF levels assayed post-MI, as described herein. The invention also provides a comparing device such as a computer for accessing the database and/or processing the query. The invention also provides a system comprising a database and at least one computer to access and/or operate the database. A or the computer may also be used to process or administer the querying of the database with the feature data of the patient to be tested. The database may be stored centrally, for instance in a server, or may be retained in the lab or field equipment used to assay the levels of VEGFB, THBS1 and/or PGF, or in a computer associated with said equipment, as discussed below. The computer may also be located centrally with the server or remotely, for instance in an intermediate lab, or located in field equipment.

A kit, which may include lab or field equipment for assaying a sample from the patient is also disclosed herein. The equipment may comprise the database or may simply comprise a display or readout of the results of the analysis. The equipment may have the ability to contact the database remotely, for instance via an internet network or the internet, whether by wire or by wireless broadcast.

Also disclosed herein is a method of obtaining feature data from a patient, by assaying VEGFB, THBS1 and PGF levels and preferably associating the level data with a patient, for instance by using a patient identifier, such as a code.

Disclosed herein is a method of receiving feature data for the VEGFB, THBS1 and PGF levels from the patient to compare it with the feature data in the database. The data may be processed by a receiver or transmitted to a processor.

Thus, disclosed herein is a method of processing feature data for the VEGFB, THBS1 and PGF levels from the patient and comparing it with the feature data in the database. The likely prognosis for the patient may be outputted by the processor in dependence upon the result of the comparison and may, optionally, be transmitted to a separate computer and/or the kit or equipment discussed above, via a network, the internet and by wire or wireless transmission.

The database may also be stored on a carrier medium, such as a disk or memory device. Thus, disclosed herein is a carrier medium comprising a database arranged to cause a computer to determine the likelihood of a patient developing HF post-MI when queried with feature data on VEGFB, THBS1 and PGF levels from a patient.

The levels of VEGFB, THBS1 and PGF may be considered to be values or ratios and do not necessarily have to be volumes or mass per unit volume and so forth.

Preferably, the reference sample is from a patient in a similar demographic, genotypic or phenotypic group to the patient. The reference sample may be considered to be in the same demographic group as the infarcted patient if any number of the following criteria are met: sex, age, race or ethnic background, and medical history. Suitable genotypic or phenotypic control samples can be selected based on any number of suitable selection criteria, such as determining the genotype of a patient at one or more loci, in particular those known to be associated with infarcted patients, heart failure and/or ventricular remodeling.

Determining the genotype may comprise detecting the presence of an amino acid change in the sequence of the hemopexin domain of MMP-9 (Matrix Metalloproteinase 9), the presence of an amino acid change in said domain being indicative of susceptibility to said heart condition, post myocardial infarction. Preferably, the sequence that is detected comprises or encodes either a Glutamine (Gln) or an Arginine (Arg) amino acid residue at a position corresponding to position 148 of the hemopexin domain of MMP-9. Preferably, the detected sequence is SEQ ID NO. 13, which is the amino acid sequence of the hemopexin domain of MMP-9 from the at risk (susceptible) group (showing Arg at amino acid position 148), or a polynucleotide sequence encoding it. The Single Nucleotide Polymorphism (SNP), present in the coding sequence of the MMP-9 gene, is found at different frequencies in patients with good or poor prognoses for heart failure following myocardial infarction and can lead to a single amino acid change in the hemopexin domain of the transcribed and active MMP-9 protein, resulting in an electrostatic change in the site on MMP-9 that binds tissue inhibitor of metalloproteinase-1 (TIMP-1). This is significant as TIMP-1 is the foremost inhibitor of MMP-9 activity. Thus, it may be useful to determine whether the present patient has the above SNP and then, if he does, compare the levels of VEGFB, THBS1 and/or PGF (in the assayed samples of the present invention) with the levels in samples from patients also known to have the above SNP, or *vice versa*.

Preferably, the determination of a decreased risk of heart failure is relative to those infarcted reference patients having comparatively high levels of VEGFB mRNA (>-1.4), relatively low levels of THBS1 (<0) and/or relatively low levels of PGF (<-0.1) respectively. These values are expressed as log ratio (patient RNA/reference RNA). The reverse holds true for an increased risk of developing a heart condition.

The above values are derived from Table 4, although it is preferred that these may vary by at least 1 or 2%, more preferably at least 5%, more preferably at least 7%, more preferably at least 10%, more preferably at least 15%, more preferably at least 20%, more preferably at least 25%, more preferably at least 30%, more preferably at least 40%, and even up to 50%.

Since the only established biomarker of HF (Heart Failure) is pro-BNP (brain natriuretic peptide), the prognostic performance of the 3 biomarkers set disclosed here was compared with the prognostic performance of NT-pro-BNP. The prognostic performance of the plasma level of NT-pro-BNP, measured 1 day after MI, was moderate (AUC=0.63, Table 5). Therefore the set of 3 biomarkers disclosed here clearly outperformed the prognostic value of NT-pro-BNP. Nevertheless, it is preferred that BNP can also be assayed in the present method, either to increase the accuracy or confirm a determined prognosis. This BNP assayed level may be compared to a BNP basal or a reference level as discussed herein.

The nucleotide and protein sequences for pro-BNP are provided in SEQ ID Nos 10-11.

Also disclosed herein is a method of establishing a prognosis for a myocardially-infarcted patient, the method comprising:
- assaying, post infarction, a body fluid sample from the patient for levels of Vascular Endothelial Growth Factor B (VEGFB) and levels of Thrombospondin-1 (THBS1) and/or Placental Growth Factor (PGF);
- comparing the levels VEGFB, THBS1 and PGF with the corresponding levels of VEGFB, THBS1 and/or PGF from a reference sample, said reference sample having a known clinical outcome; and
- determining the prognosis for said patient based on said comparison.

The levels of Thrombospondin-1 (THBS1) and/or Placental Growth Factor (PGF) are preferably low plasma levels.

Also provided is a method of establishing a prognosis for a myocardially-infarcted patient, the method comprising:
- assaying, post infarction, a body fluid sample from the patient for levels of Vascular Endothelial Growth Factor B (VEGFB) and levels of Thrombospondin-1 (THBS1) and Placental Growth Factor (PGF);
- comparing the levels VEGFB, THBS1 and PGF with a control; and
- determining the prognosis for said patient based on said comparison.

This may be achieved by comparing the levels VEGFB, THBS1 and PGF with the corresponding level of VEGFB, THBS1 and PGF in a reference sample;
wherein at least one of the following indicates an increased likelihood of an unfavorable prognosis for said patient:
- lower levels of VEGFB in the assayed patient sample compared to the VEGFB control level;
- higher levels of THBS1 in the assayed patient sample compared to the THBS1 control level; and
- higher levels of PGF in the assayed patient sample compared to the PGF control level.

Also provided is a method of establishing a prognosis for a myocardially-infarcted patient, the method comprising:
- assaying, post infarction, a body fluid sample from the patient for levels of Vascular Endothelial Growth Factor B (VEGFB) and levels of Thrombospondin-1 (THBS1) and Placental Growth Factor (PGF); and
- questioning a previously built statistical program (also called "classifier") with the levels of VEGFB, THBS1 and PGF;
wherein
- high level of VEGFB and low levels of THBS1 and PGF are associated with increased likelihood of developing heart failure; and
- the classifier will dictate whether the patient has an increased likelihood of developing heart failure.

The levels of Thrombospondin-1 (THBS1) and/or Placental Growth Factor (PGF) are preferably low plasma levels.

The unfavorable prognosis is preferably that the patient has an increased likelihood of suffering a said heart condition.

Also disclosed herein is a method of determining the likelihood of a myocardially-infarcted patient developing a heart condition, comprising the above steps.

Also disclosed herein is a method of identifying myocardially-infarcted patients having a reduced risk of developing a heart condition, comprising:
- assaying, post infarction, a body fluid sample from the patient for levels of Vascular Endothelial Growth Factor B (VEGFB), Thrombospondin-1 (THBS1) and Placental Growth Factor (PGF);
- comparing the levels VEGFB, THBS1 and PGF with the corresponding levels of VEGFB, THBS1 and/or PGF from a reference sample, said reference sample having a known clinical outcome; and
- determining whether the patient has a reduced risk based on said comparison.

According to another aspect, the present invention provides a method of identifying myocardially-infarcted patients having a reduced risk of developing heart failure, comprising:
- assaying, post infarction, a body fluid sample from the patient for levels of Vascular Endothelial Growth Factor B (VEGFB), Thrombospondin-1 (THBS1) and Placental Growth Factor (PGF);
- comparing the levels VEGFB, THBS1 and PGF with the corresponding levels of VEGFB, THBS1 and/or PGF from a control; and
- determining whether the patient has a reduced risk based on said comparison.

This method may be amended to include comparing the levels VEGFB, THBS1 and/or PGF with the corresponding level of VEGFB, THBS1 and/or PGF in a reference; wherein at least one of the following indicates a decreased likelihood of said patient suffering from said heart condition:
- higher levels of VEGFB in the assayed patient sample compared to the VEGFB control level;
- lower levels of THBS1 in the assayed patient sample compared to the THBS1 control level; and/or
- lower levels of PGF in the assayed patient sample compared to the PGF control level.

This method may be amended to include:
- questioning a previously built statistical program (also called "classifier") with the levels of VEGFB, THBS1 and PGF;
wherein
- high level of VEGFB and low levels of THBS1 and PGF are associated with a decreased likelihood of developing said heart condition; and
- the classifier will dictate whether the patient has a decreased likelihood of developing said heart condition.

Disclosed herein is a method of screening myocardially-infarcted patients for patients to assess the risk that each patient may have of developing a heart condition. This may be an increased or reduced risk.

The methods of the invention and as disclosed herein correlate the measurement of three biomarkers, with a better clinical outcome after MI. Most preferably, the first biomarker is VEGFB and if the level thereof is high at day 1 post-MI, then this patient has a more favorable clinical outcome after MI. Most preferably, the second biomarker is THBS1 and if the level thereof is low at day 1 post-MI, then this patient has a more favorable clinical outcome after MI. Most preferably, the third biomarker is PGF and if the level thereof is low at day 1 post-MI, then this patient has a more favorable clinical outcome after MI.

It will be appreciated that the present methods are useful for establishing a prognosis in patients with MI, by correlating a combined assessment of multiple biomarkers, which, depending on their levels, can indicate a better clinical outcome after MI.

The invention may also be used in a personalized medicine setting, for example in a method of providing or improving a patient's therapeutic strategy following MI, based upon identifying those patients at risk of developing a heart condition. This may be through the analysis of blood cell mRNA levels or plasma protein levels of VEGFB, THBS1 and/or PGF.

Diagnostic kits for use in the methods disclosed herein are readily available for THBS1 and PGF, such as those available from R&D Systems. Inc. However, for VEGFB, it was necessary to construct our own diagnostic kit, as discussed below. Indeed, the only commercially available VEGFB kit (from USCNLIFE, VEGFB E0144h) was not sensitive enough to detect low VEGFB plasma levels. Using enhanced chemiluminescence as the detection method and an amplification step with biotin-streptavidin, the detection limit of our kit was 10 pg/mL whereas that of USCNLIFE kit, which uses a classical colorimetric detection, was found to be around 100 pg/mL. Therefore, disclosed herein is a method for assaying VEGFB levels in a sample, comprising:
(a) contacting the sample with at least one capture reagent immobilized to a support to form an immobilized capture reagent-sample complex;
(b) separating the sample from the at least one immobilized capture reagent;
(c) contacting the immobilized capture reagent-sample complex with a secondary antibody specific for VEGFB and optionally contacting the secondary antibody with a tertiary antibody specific for the secondary antibody;
(d) contacting the secondary or tertiary antibody with a binding molecule, such as streptavidin, conjugated to detection means; and
(e) measuring the level of the secondary or tertiary antibody bound to the capture reagents using the detection means.

The capture reagent may be an antibody, most preferably one that recognizes the same epitope as antibody mouse monoclonal clone 58013 against human VEGFB, said monoclonal antibody preferably binding specifically to VEGFB 167 and/or VEGFB 186.

The secondary antibody may be an antibody that recognizes the same epitope as antibody goat polyclonal that binds specifically to VEGFB 167 and/or VEGFB 186. The tertiary antibody may be a biotin-conjugated antibody specific for the secondary antibody, for instance a donkey anti-goat Ab. Preferably, the detection means comprises an alkaline phosphatase activity.

Disclosed herein is a method for assaying VEGFB levels in a sample, comprising:
(a) contacting and, optionally incubating, the sample with a capture reagent immobilized to a solid support, wherein the capture reagent is an antibody that recognizes the same epitope as antibody mouse monoclonal clone 58013 against human VEGFB, said monoclonal antibody binding specifically to VEGFB 167 and VEGFB 186 to form an immobilized capture reagent-VEGFB complex;
(b) separating the sample from the immobilized capture reagents;
(c) contacting the immobilized capture reagent-VEGFB complex with a secondary antibody, wherein the secondary antibody is an antibody that recognizes same epitope as antibody goat polyclonal that binds specifically to VEGFB 167 and VEGFB 186;
(d) contacting the secondary antibody with a tertiary antibody, wherein the tertiary antibody is a biotin-conjugated donkey anti-goat antibody specific for the secondary antibody;
(e) contacting the tertiary antibody with streptavidin conjugated to alkaline phosphatase;
(f) measuring the level of VEGFB 186 or VEGFB 167 bound to the capture reagents using a detection means for the detectable antibody.

Additionally, we disclose herein an ELISA kit to measure levels of VEGFB 186 and VEGFB 167 in biological fluids and its use as a diagnostic tool to identify the patients at risk of developing HF after MI.

The kit preferably comprises;
(a) at least one capture reagent immobilized to a support;
(b) a secondary antibody specific for VEGFB 186 and/or VEGFB 167;
(c) optionally, a tertiary antibody specific for the secondary antibody;
(d) a binding molecule, such as streptavidin, conjugated to detection means; and
(e) means for measuring the level of the secondary or tertiary antibody bound to the capture reagents using the detection means.

As above, the capture reagent may be an antibody, most preferably one that recognizes the same epitope as antibody mouse monoclonal clone 58013 against human VEGFB, said monoclonal antibody preferably binding specifically to VEGFB 167 and/or VEGFB 186. The secondary antibody may be an antibody that recognizes the same epitope as antibody goat polyclonal that binds specifically to VEGFB 167 and/or VEGFB 186. The tertiary antibody may be a biotin-conjugated antibody specific for the secondary antibody, for instance a donkey anti-goat Ab. The detection means may comprise an alkaline phosphatase activity.

Said biological sample may, preferably, be isolated from a human subject and may be plasma or serum. It is also preferred that the immobilized capture reagents are coated on a microtiter plate. Preferably the detection is amplified by a chemiluminescent reagent. Purified human VEGFB 167 may be provided as an antigen standard.

We compared our VEGFB kit with the only commercially available VEGFB kit we found (USCNLIFE VEGFB E0144h). Our kit is more sensitive and therefore allows to measure VEGFB in more patients than the USCNLIFE kit.

According to a further aspect, the present invention provides a method of identifying myocardially-infarcted patients having a decreased risk of developing heart failure, comprising:
- assaying, post infarction, a body fluid sample from the patient for levels of Vascular Endothelial Growth Factor B (VEGFB), Thrombospondin-1 (THBS1) and Placental Growth Factor (PGF); and
- questioning a previously built statistical program (also called "classifier") with the levels of VEGBF, THBS1 and PGF;
wherein
- high level of VEGFB and low levels of THBS1 and PGF are associated with a decreased likelihood of developing heart failure; and
- the classifier will dictate whether the patient has a decreased likelihood of developing heart failure.

We have a large database of acute MI patients with more than 20 clinical parameters and 4-months and 1-year follow-ups. Among other clinical parameters, the ejection fraction (EF) of the heart, which represents the capacity of the heart to pump blood into peripheral arteries, was measured by echocardiography the day of infarction, 4 months later and 1 year later. It is assumed that patients having an EF at 4-months ≤ 40% suffer from remodeling whereas patients having an EF at 4-months > 40% are recovering normally. Several protocols performed in our laboratory used the database to identify early biomarkers of the occurrence of HF after MI through different approaches. The biomarkers identified by two fundamentally different approaches were combined and the combination of the most predictive biomarkers was defined as the "prognostic set".

The first approach involved DNA microarray technology. Biosignatures or gene expression profiles of circulating blood cells were analyzed from blood samples withdrawn the day of MI. This technology allowed the identification of differentially regulated genes between two groups of patients with extreme phenotypes, i.e. one group of patients having a favorable clinical outcome after MI (high EF group, EF>40%) and one group having an unfavorable outcome after MI (low EF group, EF≤40%). We have characterized the biosignatures of blood cells from 32 patients, 16 from each high EF and low EF group. Using the SAM algorithm (Statistical Analysis of Microarrays) and a fold-change of 1.3, 525 genes were found differentially expressed between the 2 groups of patients (False Discovery Rate of 24.5%). Among these genes, 9 had a significant prognostic value for HF.

The second approach was based on a bioinformatic characterization of a protein-protein interaction network of angiogenesis in human MI. Indeed, angiogenesis is one of the beneficial healing processes that take place in the heart after MI and a defect in angiogenesis can lead to HF. The network was built with annotated protein-protein interactions from the Human Protein Reference Database. This global network consisted of 556 nodes (i.e. proteins) and 686 edges (i.e. interactions). After subsequent network-based and gene expression analyses, 38 network-derived genes showed a significant prognostic value. Interestingly, the combination of the gene expression-based classification models with the network-based classification models yield to a reduced number of candidate biomarkers with a greatly improved prognostic value than each approach considered separately. The area under the curve (AUC), which represents the prognostic capacity of the biomarkers, was between 0.56 and 0.72 for the gene expression-based classification model, and between 0.56 and 0.73 for the network-based classification model. When the two models were combined, a set of 3 biomarkers with and AUC of 0.82 (i.e. with a strong prognostic value for the occurrence of HF) was implemented: this set is called here the "prognostic set". These 3 biomarkers were: thrombospondin-1 (THBS1), placental growth factor (PGF or PlGF), and Vascular Endothelial Growth Factor B (VEGFB). While THBS1 has anti-angiogenic properties, VEGFB and PGF owe their pro-angiogenic capacities to stimulation of the growth and multiplication of vascular endothelial cells.

Then, we verified that the differences in gene expression observed at the mRNA level by microarrays were effective at the protein level. For this purpose, plasma levels of the 3 biomarkers were measured by enzyme-linked immunosorbent assay (ELISA). These experiments attested that protein levels of THBS1 and PGF were significantly distinguishable between patients with high and low EF.

Since VEGFB ELISA kits commercially available were not found sensitive enough to detect VEGFB in our plasma samples, we designed our own kit which allows the quantification of VEGFB in biological fluids such as human plasma.

By "favorable outcome," it will be understood that this means a lower risk of the patient going on to develop a heart condition, such as Heart Failure and/or suffer from Left Ventricular remodeling. It is believed that Right Ventricular Remodelling is less relevant, so this is not preferred.

By "unfavorable outcome," it will be understood that this means a higher risk of the patient going on to develop a heart condition, such as Heart Failure and/or suffer from Left Ventricular remodeling.

It will also be understood that whilst the risks of Heart Failure and Left Ventricular Remodeling are associated, these are separate conditions and, therefore, a patient could suffer one, but not the other. Therefore, increased risk of either Heart Failure or Ventricular Remodeling is unfavorable.

It will be appreciated that the RNA sequences given in the sequence listing comprise Thymine (T) as this is how they are represented on the NCBI website. In each case, it is clear that replacement of T with Uracil (U) is contemplated.

### BRIEF DESCRIPTION OF THE DRAWINGS

Fig. 1 is an illustration of the microarrays data interpreted with SAM algorithm.
Fig. 2 shows the protein-protein interaction network of angiogenesis in human MI.
Fig. 3 illustrates the strategy used for the combined analysis of the gene expression-based classification models with the network-based classification models.
Fig. 4 shows a heat-map illustrating the differences in the expression (microarrays) of the biomarkers among patients with high (H) and low (L) ejection fraction.
Fig. 5 shows quantile-quantile plots illustrating the relationship between the ejection fraction and the expression of the biomarkers assessed by microarrays and ELISA.
Fig. 6 shows scatter-plots illustrating the relationship between the ejection fraction and the expression of the biomarkers assessed by microarrays and ELISA.
Fig. 7 represents the evolution of VEGFB plasma levels between the day of infarction (day 0) and the day after (day 1). Whereas plasma VEGFB decreases between day 0 and day 1 in patients with low EF (-10%), patients with high EF have increasing VEGFB levels (+15.4%).
Fig. 8A Shows expression values of VEGFB using quantitative PCR and microarrays for high EF and low EF patients
Fig. 8B shows the significant correlation observed between VEGFB expression and ejection fraction.
Fig. 9 shows VEGFB levels between high and low EF groups at day 0, day 1 and day 2 after MI.
Table 1 is a summary of the predictive performances of classification models based on mRNA levels of VEGFB, THBS1 and PGF.
Table 2 is a summary of the conclusions reached in the experiments underling the present invention.
Table 3 is a summary of the statistics performed to compare the levels of the VEGFB, THBS1 and PGF between patients with high EF and patients with low EF.
Table 4 is a summary of the statistics of the comparison between mRNA levels of the 3 biomarkers in the two groups.
Table 5 is a summary of the predictive performance of NT-pro-BNP.
Table 6 is a list of 28 angiogenic genes differentially expressed between high and low EF groups.
Table 7 shows prediction performances using two machine learning models.

### DETAILED DESCRIPTION

Heart Failure (HF) is the major complication of myocardial infarction (MI). Recent data showed that 63% of the patients develop HF in the 6 years following MI. Angiogenesis is a key phenomenon involved in the repair of the myocardium after MI. Angiogenesis is tightly regulated by a balance being governed by a large number of angiogenic factors, some being pro- and others being anti-angiogenic. A deregulation of this balance can lead to inappropriate angiogenesis and can set the stage for the development of HF after a MI episode.

Recent reports clearly indicate that the early identification of patients prone to suffer from HF after MI may significantly improve the tailoring of the therapeutic strategies to the individual patient ("personalized medicine"). Like many other cardiovascular disorders, HF is a multifactorial disease. Thus the use of single biomarkers to predict the occurrence of HF after MI has limited value. The multiplication of prognostic biomarkers may therefore be of interest to finely predict HF.

First, we hypothesized that a set of prognostic biomarkers could be identified through the analysis of the biosignatures of circulating blood cells. Second, we hypothesized that a protein-protein interaction network-based approach may also have the potential to highlight prognostic biomarkers of HF. And third, we tested whether the combination of the data obtained by these two independent approaches would allow achieving a higher level of prognosis than each approach taken separately.

For this purpose, we selected two groups of patients (n=16 per group) with MI, one group of patients having a favorable outcome after MI (Ejection Fraction (EF) >40%) and one group of patients having an unfavorable outcome (EF≤40%). This strategy to select "extreme phenotypes" was chosen to increase the chances of finding differentially expressed genes between the two groups without the need of a large sample size. RNA was extracted from whole cells isolated from the peripheral blood of these patients. Biosignatures were determined by microarray profiling. After several normalization, filtering and statistical procedures, a set of 525 genes were found to be differentially expressed between the two groups of patients (fold-change 1.3; false discovery rate 24.5%). Among those, a cluster of 47 genes with moderate prognostic value was identified by classification models with a maximum AUC of 0.72. Further filtering of these genes led to a cluster of 9 genes with equivalent prognostic value (AUC 0.68).

In an attempt to increase the strength of the prediction afforded by genes retrieved from microarray experiments, and considering the importance of angiogenesis in the repair of the heart after MI, a protein-protein interaction network of angiogenesis in human MI was drawn. This network was assembled by extracting genes known to be involved in this process and corresponding (curated) protein-protein interactions from public databases. Clustering analysis of this network reported a module significantly associated with cell growth and growth regulation. Within this cluster, 38 genes were found to be significantly differentially expressed between the EF classes. Different, independent classifiers built with these 38 genes reported a moderate prognostic value (max. AUC = 0.73), equivalent to that obtained from microarrays. Interestingly, further filtering of these genes (correlation-based feature selection) yielded a set of 3 genes with a stronger prognostic value (AUC = 0.82 using an instance-based learner, Table 1). These 3 genes, VEGFB, THBS1 and PGF, are viewed here as a new "prognostic set" of biomarkers of HF. Differential expression of these 3 genes was validated by quantitative PCR. In addition, plasma levels of the 3 biomarkers were measured.

In one finding, we showed that patients able to mount a significant response to MI, characterized by high mRNA or plasma protein levels of Vascular Endothelial Growth Factor B (VEGFB) and low plasma levels of Thrombospondin-1 (THBS1) and Placental Growth Factor (PGF or PlGF), have a low susceptibility to develop HF and/or undergo Ventricular Remodeling. Measurement of the plasma levels of these three biomarkers can therefore serve as a useful tool for predicting the occurrence of HF and/or Ventricular Remodeling after MI.

**Table 1. Predictive performance of classification models based on mRNA levels of the 3 biomarkers.**

| **Input type** | **Classification model** | **Typical accuracy* (%)** | **AUC*** |
|---|---|---|---|
| SAM-based biomarkers only | K* | 65 | 0.63 |
| Network-based biomarkers | K* | 84 | 0.82 |
| SAM-based biomarkers only | SVM | 68 | 0.68 |
| Network-based biomarkers | SVM | 75 | 0.75 |

| | | | |
|---|---|---|---|
| * Based on leave-one-out cross-validation. | | | |

Potential biomarkers used: SAM-derived (9 genes) and network-based (3 genes). AUC: Area under the (ROC) curve; SVM: Support Vector Machine.

Therefore, we propose a new strategy to identify the patients at risk of developing HF after MI, based on the measurement of a cluster of 3 biomarkers, VEGFB, THBS1 and PGF. These measurements can be made from RNA extracted from blood cells or from plasma levels of the corresponding proteins.

Table 2 summarizes the particular findings behind the present invention.

**Table 2. Association between the mRNA and plasma levels of the 3 biomarkers and the EF.**

| | **High EF (favorable outcome)** | | **Low EF (unfavorable outcome)** | |
|---|---|---|---|---|
| | **mRNA** | **Plasma** | **mRNA** | **Plasma** |
| **VEGFB** | High | High | Low | Low |
| **THBS1** | Low | Low | High | High |
| **PGF** | Low | Low | High | High |

| | | | | |
|---|---|---|---|---|
| mRNA levels are measured by microarrays in blood samples harvested the day of MI Plasma levels are measured by ELISA one day after MI. | | | | |

All references cited herein are hereby incorporated in their entirety to the extent that they do not conflict with the present invention.

The invention will now be described in further detail in the accompanying non-limiting examples.

### EXAMPLES

### EXAMPLE 1

### PATIENTS AND METHODS

### Patients

Patients with acute MI were treated with primary percutaneous coronary intervention. Acute MI was defined by the presence of chest pain < 12 hours with significant ST segment elevation and positive cardiac enzymes. Blood samples were obtained at the time of mechanical reperfusion (for microarrays and quantitative PCR analyses) and the day after MI (for plasma levels determination). All patients signed an informed consent.

### Microarrays

To increase our chances to detect relevant biomarkers in the context of ventricular remodeling, we selected two groups of patients having "extreme" phenotypes after MI, namely patients that evolved favorably after infarction (EF≥45%, average 61%) and patients that evolved unfavorably (EF≤40 %, average 33%). Each group contained 16 patients.
Total RNA was extracted from whole blood cells by the PAXgene™ technology. Blood withdrawn in PAXgene™ blood RNA tubes (PreAnalytix®, BD Europe, Erembodegem, Belgium) was stored at -20°C until RNA extraction. Extraction was performed with the PAXgene™ Blood RNA kit (Qiagen, Courtaboeuf, France) according to the manufacturer's instructions. RNA quantity was measured using the ND-1000 spectrophotometer (NanoDrop^{®} Technologies, Wilmington, USA). RNA quality was assessed using the 2100 Bioanalyzer^{®} apparatus (Agilent Technologies, Massy, France) with the RNA 6000 Nano chips. Only high quality RNA (OD₂₆₀/OD₂₈₀ > 1.9 and OD₂₆₀/OD₂₃₀ > 1.7) and undegraded RNA was considered for further analysis.

A common reference RNA (Universal Human Reference RNA, Stratagene Europe, Amsterdam, The Netherlands), a mixture of RNA from 11 cell lines was used in conjunction with patient's RNA in all following steps in order to provide an internal reference standard for comparisons of relative gene expression levels across arrays.

Messenger RNAs were amplified using the Amino Allyl MessageAmp™ kit (Ambion^{®}, Cambridgeshire, United Kingdom) according to the manufacturer's protocol, starting with one µg of total RNA. Five µg of each amino allyl aRNA were labeled with Cy3 or Cy5 (Amersham, Buckinghamshire, United Kingdom). Dye coupling to amino allyl aRNA was measured using the ND-1000 NanoDrop^{®} spectrophotometer. Dye coupling yield >5% was a prerequisite for further analysis. 750 ng of each amino allyl aRNA labeled Cy3 or Cy5 (reference RNA or donor RNA) were combined and hybridized on pangenomic oligonucleotide microarrays containing 25,000 genes (Genomic Platform, Illkirch, France). Four microarrays per patient were hybridized and a dye-swap was performed (2 microarrays patient-Cy3/reference-Cy5 and 2 microarrays patient-Cy5/reference-Cy3). Hybridization steps were performed using the Agilent Technologies system. Briefly, RNA was fragmented with a fragmentation buffer before mixing with a hybridization buffer. Microarrays were blocked with 50 mM ethanolamine in 50 mM borate buffer pH= 9.0. Agilent's hybridization chambers and rotating oven were used for hybridization at 60°C for 17 h at 4 rpm. Microarrays were washed for 10 min in 6X SSC, 0.005% Triton X-102, for 5 min in 0.1X SSC, 0.005% Triton X-102, and were then dried by centrifugation before scanning using an Axon 4000B microarray scanner and the GenePix Pro 6^{®} software (Molecular Devices, Berks, UK). Self photomultiplicator gain adjustment and 0.1% saturated spots were allowed during scanning.

Spot finding and raw-data quantification of all four microarrays for each patient were performed in a batch analysis using the MAIA^{®} freeware (Institut Curie, France). This software assigns each spot with nine quality parameters that allow for determination of "good quality spots" among the four microarrays. Only good spots are kept for further analysis. A Lowess non-linear normalization step was performed with the Acuity^{®} software (Molecular Devices) to compensate for uneven Cy3-Cy5 distribution. Normalized log ratio of Cy3/Cy5 was used in subsequent steps. A filtering step was then performed to remove genes that were not present in at least three microarrays out of four. The quality and reproducibility of each of the four microarrays per patient were evaluated using ANOVA, correlation coefficients and Self Organizing Maps drawn with the Acuity^{®} software. Data are stored in the Web-based Microarray Data manager MEDIANTE.

Before statistical analysis, genes not present in at least 50% of the patients were filtered out. Supervised analysis was performed using two complementary approaches. The first approach involved the Significance Analysis of Microarrays (SAM) software which correlates gene expression with an external variable such as EF value. Two-class unpaired test was used. Gene missing-values imputation was performed via a K-Nearest Neighbour algorithm using 10 neighbours.

### Protein-protein interaction network

A set of core genes, known to be associated with angiogenesis in myocardial infarction, was retrieved from the Entrez-Gene database; query: "human AND heart AND angiogenesis AND myocardial AND infarction". Annotated protein-protein interactions associated with these core genes were retrieved from the Human Protein Reference Database (HPRD).

A network clustering analysis was implemented to identify potential functional network modules. Clusters were identified by the (Cytoscape plug-in) MCODE network clustering algorithm.

### Biochemical assays for THBS1, PGF and NT-pro-BNP

Plasma levels of THBS1 and PGF were measured in samples from 46 patients by ELISA using the Quantikine DTSP10 and DPG00 kits, respectively (R&D Systems, Oxon, UK). Detection limits of the assays were 0.35 ng/mL for THBS1 and 7 p/mL for PGF. Plasma level of pro-BNP (N-Terminal -pro-BNP, NT-pro-BNP) was measured using the Elecsys 2010 immunological device (Roche Diagnostics, Meylan, France). Detection limit of the assays was 20 pg/mL.

### Set up of the VEGFB diagnostic kit

A sandwich ELISA was developed to detect VEGFB 167 and VEGF-B 186. Microtiter plates (Lumitrac 600, Greiner, Belgium) are coated with 100µl of mouse anti-VEGF-B monoclonal antibody (2µg/ml in PBS, MAB751, R&D systems, UK) overnight at 4°C. After three washings, plates are blocked for 1 hour with 300µl of 5% BSA-PBS at 500 rpm and room temperature. A standard curve is produced from 2000 pg/ml to 15.6 pg/mL with human VEGFB 167 (751-VE, R&D Systems) in 1% BSA-PBS. After blocking, plates are washed three times and incubated for 2 hours with 100µl of plasma, blank or standards at 500 rpm and room temperature. After three washes, 100µl of goat polyclonal VEGF-B antibody (400ng/ml in 1% BSA-PBS, AF751, R&D Systems) are added to each well and plates are incubated for 1 hour at 500 rpm and room temperature. After three washes, 100µl of biotin conjugate donkey anti-goat antibody (1:27500 in 1% BSA-PBS, 705-065-147, Jackson, USA) are added to each well and plates are incubated for 1 hour at 500 rpm and room temperature. After three washes, 100µl of streptavidin conjuged to alkaline phosphatase (2µg/ml in 1% BSA-PBS, 016-050-084, Jackson) are added to each well and plates are incubated for 1 hour at 500 rpm and room temperature. Plates are washed four times with Tris-Buffered Saline added with Tween 20 (pH 7.5) before incubation with 100µl per well of Lumiphos 530TM (Lumigen, USA) for 30 minutes at 500 rpm, room temperature and protected from light. Chemiluminescence is detected using a Polarstar Optima (BMG Labtech, Paris, France).

### Statistical analysis

For each set of inputs, different standard statistical and machine learning classifiers were evaluated, e.g. Prediction Analysis for Microarrays (PAM), Support Vector Machine (SVM) and the K* techniques. The K* is an instance-based model that classifies a new sample based on the class information provided by its most relevant (or nearest) neighbors in a training dataset. K* applies an entropy-based distance measure to estimate the neighborhood set. Models were implemented with global blend = 20, and average column entropy curves for estimating missing values.

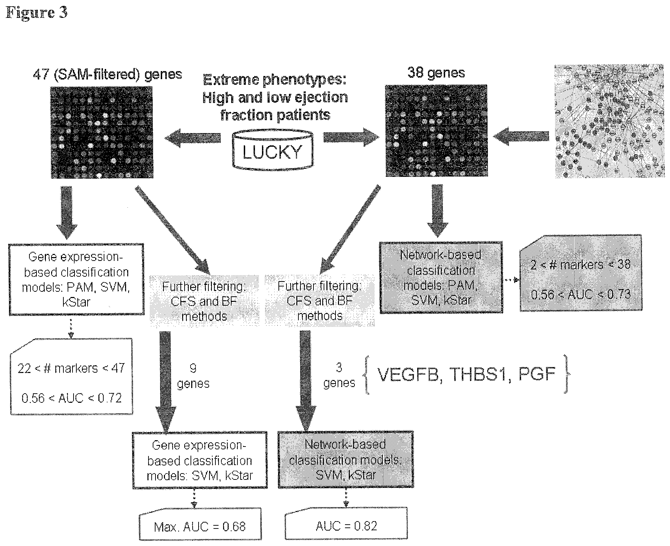

Further filtering on the network-based gene data was implemented with the correlation-based feature selection (CFS) algorithm using the "best first search" (BF) strategy (Fig. 3). The CFS is a filter feature selection method that finds subsets of features (i.e. genes) that maximizes gene-class correlation while minimizing gene-gene correlation. Filter feature selection methods are implemented independently of any classification model. The BF strategy was based on a greedy hill-climbing augmented with a subset backtracking.

Classification evaluation results were estimated using the leave-one-out cross-validation (LOO) strategy, as well as 10-fold cross-validation. The estimated areas under the curves (AUC) of the cross-validated ROC (receiver operating characteristic curve) were used to summarize the estimated classification performance of the classifiers.

Statistical differences between EF groups (on the basis of each of the biomarkers) was implemented through Student's t test, and corroborated with non-parametric tests. Correlations between these biomarkers and the EF values were estimated with standard Pearson coefficients (Table 2).

### Software

Machine learning models implementation and statistical evaluation were performed with the Weka (v. 3.4) data mining platform. Hierarchical clustering was implemented using unweighted pair-group method with arithmetic averages and correlation coefficients. Clustering visualization was performed with GEPAS. Statistical significance tests, Pearson correlation values, and graphical plots were generated with the Statistica package (v. 6.0).

### RESULTS

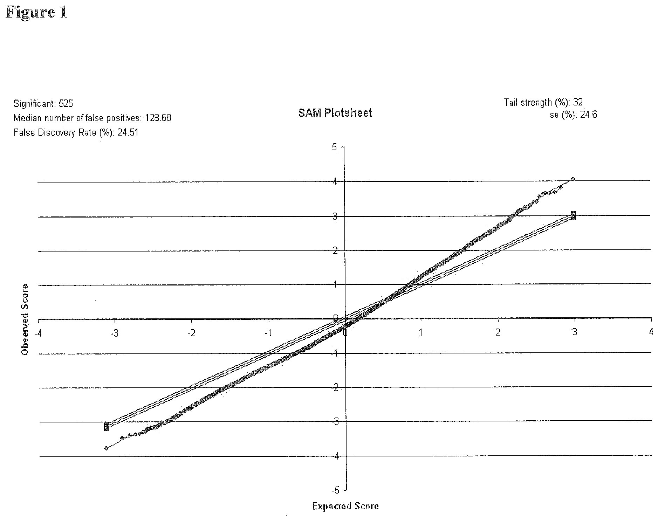

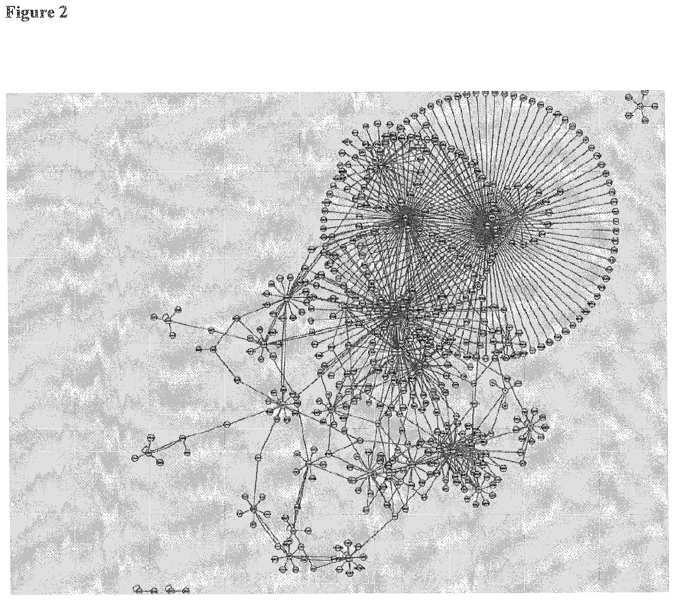

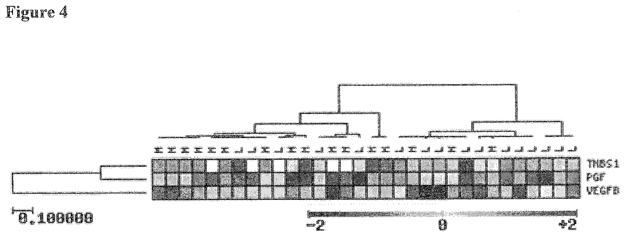

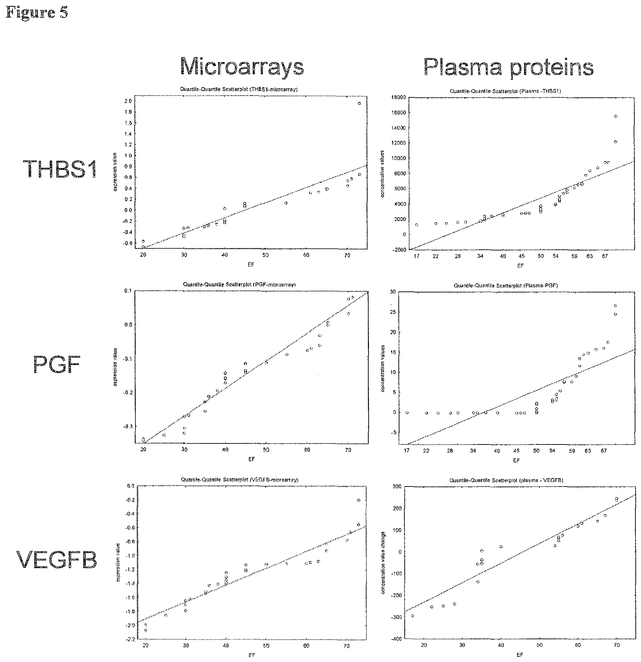

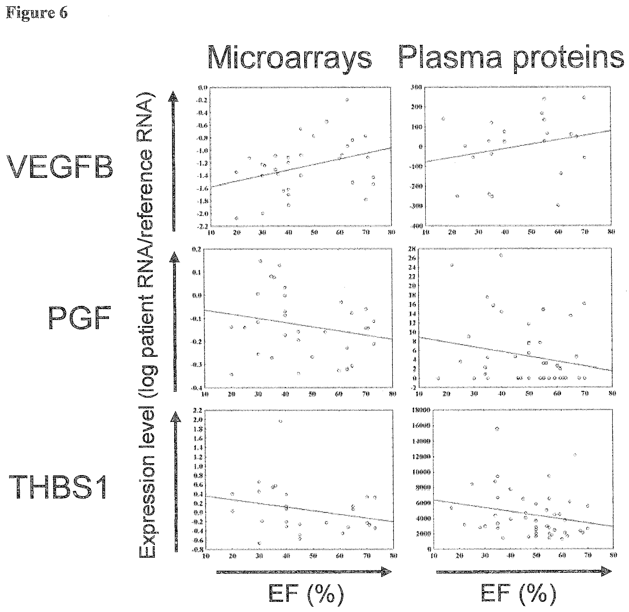

1. Statistical Analysis of Microarrays (SAM) plot showing that 525 genes are differentially expressed between low and high EF groups (Figure 1). A threshold for fold-change of 1.3-fold was selected and a FDR of 24.5% was obtained. Red dots represent genes up-regulated in the low EF group, green genes up-regulated in the high EF group, and black dots represent genes whose fold-change is < 1.3 between the two groups.
2. Protein-protein interaction network of angiogenesis in human MI (Figure 2). The resulting network consisted of 556 nodes (proteins) and 686 edges (interactions). Network clustering analysis consistently highlighted the existence of a network cluster (53 proteins) with a highly significant over-representation of (Gene Ontology) biological processes relating to cell growth and growth regulation.
3. Figure 3 shows that combined analysis of the gene expression-based classification models with the network-based classification models allowed the identification of 3 genes with higher prognostic value (max. AUC = 0.82) than gene expression-based classification models alone (0.56<AUC<0.72) or network-based classification models alone (0.56<AUC<0.73). The highest prognostic performances (based on the 3 genes) obtained to date have been obtained with the instance-based learning model K*.
4. A heat-map illustrates the differences in the expression (obtained by microarrays) of the biomarkers among patients with high (H) and low (L) ejection fraction (Figure 4). Colors (red, pink, light blue, dark blue) show the range of expression values (high, moderate, low, lowest). White color indicates undetectable values. VEGFB is clearly more expressed in the group of patients having a high EF whereas THBS1 and PGF are more expressed in the low EF group.
5. Quantile-quantile plots (Figure 5) and scatter-plots (Figure 6) illustrating statistical dependencies between the ejection fraction and the expression of the biomarkers assessed by microarrays and ELISA. The linear relation shown suggests that these variables follow similar data distributions. Results from Student's t-test and linear coefficient correlation corresponding to these plots are summarized in Table 3. VEGFB is positively correlated with the EF, whereas THBS1 and PGF are negatively correlated with the EF. Concordant results are obtained with microarrays and ELISA for THBS1 and PGF. VEGFB mRNA level was found significantly higher in low EF patients than in low EF patients by the microarray technique.

**Table 3**

| **Gene** | **Microarrays** | **Plasma proteins** |
|---|---|---|
| THBS1 | t = 2.4, p = 0.02 | t = 2.1, p = 0.04 |
| | r = -0.3, p = 0.2 | r = -0.2, p = 0.1 |
| PGF | t = 2.8, p = 0.01 | t = 2.2, p = 0.04 |
| | r = -0.2, p = 0.2 | r = -0.2, p = 0.2 |
| VEGFB | t = 3.1, p = 0.004 | t = -1.3, p = 0.20 |
| | r = 0.3, p = 0.05 | r = 0.2, p = 0.3 |

| | | |
|---|---|---|
| t: t statistic r: linear correlation coefficient Statistics performed on mRNA levels measured by microarrays in blood samples harvested the day of MI and plasma levels measured by ELISA one day after MI. | | |

6. Descriptive statistics of the mRNA levels of the 3 biomarkers in the two groups of HF patients showing the higher level of VEGFB mRNA and the lower levels of THBS1 and PGF mRNA in high EF patients compared to low EF patients (Table 4). Also provided in this table are theoretical thresholds for mRNA levels associated with either a high EF (>40%) or a low EF (≤40%) as determined from figures 5 and 6. For instance, in the population of HF patients used in this study, a patient having a level of VEGFB mRNA higher than -1.4, a level of THBS1 mRNA lower than 0 and a level of PGF mRNA lower than -0.1 was more prone to have a high EF. Conversely, a patient having a level of VEGFB mRNA lower than -1.4, a level of THBS1 mRNA higher than 0 and a level of PGF mRNA higher than -0.1 was more prone to have a low EF. In other words the levels of biomarkers were compared to a reference sample after the reference sample had been calibrated against a range of clinical outcomes. It is important to mention that the combination of the 3 biomarkers rather than each biomarker alone or a combination of 2 biomarkers is more accurately associated with the EF.

**Table 4. Descriptive statistics of the mRNA levels of the 3 biomarkers in the two groups.**

| | **High EF** | | **Low EF** | | |
|---|---|---|---|---|---|
| | (favorable outcome) | | (unfavorable outcome) | | |
| | **mean ± SD (min / max)** | **Threshold** | **mean ± SD (min / max)** | **Threshold** | **T stat** |
| **VEGFB** | -1.05 ± 0.42 | > -1.4 | -1.46 ± 0.32 | < -1.4 | t=3.1 |
| | (-1.78 / -0.20) | | (-2.07/-1.09) | | p=0.004 |
| **THBS1** | -0.13 ± 0.28 | <0 | 0.29 ± 0.58 | >0 | t=2.4 |
| | (-0.57 / 0.33) | | (-0.66 / 1.97) | | p=0.02 |
| **PGF** | -0.19 ± 0.10 | < -0.1 | -0.07 ± 0.15 | > -0.1 | t=2.8 |
| | (-0.34 / -0.03) | | (-0.34 / 0.15) | | p=0.01 |

| | | | | | |
|---|---|---|---|---|---|
| t: t statistic p : probability value SD : standard deviation | | | | | |

7. Additional testing of the classifier using plasma levels of VEGBF, THBS1 and PGF measured 1 day after MI in the same 32 patients that have been used to build the classifier yielded an AUC of 0.75. This suggests that the prediction performance of the classifier is good even when using a different dataset encoding other types of measurements (e.g. plasma levels).
8. Since the only established biomarker of HF is pro-BNP, the prognostic performance of the 3 biomarkers set disclosed here was compared with the prognostic performance of NT-pro-BNP. The prognostic performance of the plasma level of NT-pro-BNP, measured 1 day after MI, was moderate (AUC=0.63, Table 5). Therefore the set of 3 biomarkers disclosed here clearly outperformed the prognostic value of NT-pro-BNP.

**Table 5. Predictive performance of NT-pro-BNP.**

| **Input type** | **Classification model** | **Typical accuracy*** **(%)** | **AUC*** |
|---|---|---|---|
| NT-pro-BNP | K* | 50 | 0.52 |
| NT-pro-BNP | SVM | 63 | 0.63 |

| | | | |
|---|---|---|---|
| * Based on leave-one-out cross-validation. AUC: Area under the (ROC) curve; SVM: Support Vector Machine. | | | |

### EXAMPLE 2

### PATIENTS AND METHODS

Patients with acute MI were enrolled in a national MI registry and treated with primary percutaneous coronary intervention. Acute MI was defined by the presence of chest pain < 12 hours with significant ST elevation and increase in creatine kinase and troponin I to greater than 2 fold upper limit of normal. Blood samples were obtained at the time of mechanical reperfusion (for RNA and plasma isolation), one day or two days after MI (for plasma). The protocol has been approved by the local ethics committee and informed consent has been obtained from all subjects.

The validation cohort of 290 MI patients was from a prospective study conducted at the University Hospitals of Leicester NHS Trust (UK). Echocardiography was carried out at discharge and 6 months after MI. LV end diastolic volume (LVEDV) was estimated using the bi-planar modified Simpson's rule from apical two and four chamber views. The degree of LV remodelling was assessed from the change in LVEDV (□EDV) between discharge and follow-up.

### Microarrays

Transcriptomic profiles of whole blood cells were obtained using oligonucleotide microarrays representing 25,000 genes. Data are available at the Gene Expression Omnibus database (www.ncbi.nlm.nih.gov/geo/) under the accession number GSE8723. Supervised analysis was performed using the Significance Analysis of Microarrays (SAM) software. Statistical significance of the over representation of Gene Ontology (GO) terms in gene sets was estimated with the DAVID database. Heat maps were drawn using the Gene Set Enrichment Analysis (GSEA) software.

### Measure of VEGFB expression

VEGFB mRNA expression in blood cells obtained the day of MI was determined by quantitative PCR. A homemade sandwich ELISA was developed to measure plasma levels of VEGFB.

### Patient classification models

Support Vector Machine (SVM) and the K* computational classification models were evaluated to test the prognostic significance of VEGFB expression levels. The SMO (sequential minimal optimization) algorithm for training SVM classifiers was implemented with the following parameters: complexity parameter C = 1.0, epsilon = 1.0E-12, exponent of polynomial kernel = 1.0. Models were implemented with global blend = 20, and average column entropy curves for estimating missing values. Classification evaluation results were estimated using the leave one out cross validation (LOO) strategy. The area under the receiver operating characteristic curve (AUC) was used to summarize the estimated classification performance of the classifiers.

### Statistical analysis

Comparisons between the means of two groups of patients were performed with two-tailed unpaired t-test for Gaussian data and Mann-Whitney test for non Gaussian data. Categorical variables were compared using the Fisher exact test. Correlation between biomarker levels and the EF class was estimated with the Spearman test. Machine learning models implementation was performed with the Weka (v. 3.4) data mining platform. Hierarchical clustering was implemented using unweighted pair-group method with arithmetic averages and correlation coefficients. Clustering visualization was performed with GEPAS. Statistical significance tests were generated with the Statistica package (v. 6.0).A P value < 0.05 was considered statistically significant.

### Results

### Patient selection and characteristics

We enrolled patients presenting with acute ST elevation MI, treated by mechanical reperfusion. For transcriptomic analyses, two groups of 16 patients with acute MI were selected based on their EF 1 month after MI. One group of patients had a preserved LV systolic function with high EF after MI (> 40%, median 63%, range 45-73), and the other group impaired LV function with low EF (≤ 40%, median 35%, range 20-40).

### Transcriptomic analysis of blood cells

Gene expression profiles of whole blood cells isolated at the time of reperfusion were obtained using 25,000 genes microarrays. Among these, 525 genes were found differentially expressed by SAM between high EF and low EF patients with a 1.3 fold change threshold and a false discovery rate of 24.5%. 226 genes were up regulated in the high EF group and 299 were up-regulated in the low EF group. Out of the 525 genes, GSEA retrieved the 50 genes most significantly associated with one or the other group of patients.

### Angiogenic genes associated with clinical outcome after MI

Following our working hypothesis that angiogenesis may play a significant role in cardiac repair after MI, we aimed to identify from the 525 genes differentially expressed between high and low EF patients those genes related to angiogenesis. For this purpose, we retrieved from the Entrez Gene database a list of 494 genes known to be related to angiogenesis in humans with the following query: "angiogenesis" AND "homo sapiens". Of the 525 differentially expressed genes, 28 were found in this set of 494 angiogenic genes: 20 up regulated in the low EF group and 8 up regulated in the high EF group (*Table 6*).

**Table 6. List of 28 angiogenic genes differentially expressed between high and low EF groups.**

| **Over**-**expressed in low EF group** | | | | | | |
|---|---|---|---|---|---|---|
| Pro angiogenic (n=15) | Fold-change | q-value (%) | | Anti angiogenic (n=6) | Fold-change | q-value (%) |
| BMX | 1.90 | 11.36 | | CLU | 1.50 | 4.97 |
| PBEF1 | 1.73 | 20.21 | | THBS1 | 1.44 | 5.88 |
| FOS | 1.66 | 4.09 | | ITGB1 | 1.34 | 25.29 |
| PFKFB3 | 1.65 | 0.00 | | MAPK14 | 1.31 | 24.72 |
| CD55 | 1.65 | 5.53 | | STAT1 | 1.31 | 25.56 |
| HIF1A | 1.63 | 0.00 | | MME | 1.31 | 20.21 |
| IL8 | 1.59 | 7.51 | | | | |
| PTGS2 | 1.55 | 11.36 | | | | |
| TGFBR1 | 1.50 | 9.87 | | | | |
| THBS1 | 1.44 | 5.88 | | | | |
| SLC2A3 | 1.40 | 0.00 | | | | |
| ERO1L | 1.32 | 28.34 | | | | |
| PLAUR | 1.31 | 5.88 | | | | |
| ADM | 1.31 | 25.06 | | | | |
| B2M | 1.30 | 28.12 | | | | |

| **Under-expressed in low EF group** | | | | | | |
|---|---|---|---|---|---|---|
| Pro angiogenic (n=4) | Fold-change | q-value (%) | | Anti angiogenic (n=4) | Fold-change | q-value (%) |
| **VEGFB** | 0.74 | 4.97 | | SOD1 | 0.70 | 11.36 |
| RHOC | 0.74 | 8.70 | | MAGED1 | 0.75 | 9.31 |
| CX3CR1 | 0.76 | 17.27 | | ANXA2 | 0.76 | 17.27 |
| ATP5B | 0.80 | 21.32 | | BAI1 | 0.76 | 17.27 |

| | | | | | | |
|---|---|---|---|---|---|---|
| **q value:** the lowest false discovery rate at which the gene is called significant (like 'p value' adapted to the analysis of a large number of genes). Note: the total number of genes in this table is 28 since thrombospondin 1 (THBS1) is both pro and anti angiogenic. | | | | | | |

A heat map drawn with these 28 genes showed that clinical outcome after MI is associated with a distinct biosignature linked to angiogenesis.

In an attempt to evaluate whether this biosignature was associated with stimulation or repression of angiogenesis, we questioned the Entrez Gene database for pro or anti angiogenic properties of the 28 angiogenic genes found differentially expressed between the 2 groups of MI patients. As shown in *Table* 6, the balance between known pro and anti angiogenic factors tends to lean toward the pro angiogenic side for low EF patients, although it can only be speculated that this is associated with stimulation of angiogenesis.

We then further narrowed down our investigations on VEGFB because: (1) among the 4 pro angiogenic genes over-expressed in the high EF group (and thus potentially implicated in the favourable remodelling of the heart), only VEGFB was retrieved by the Entrez Gene database using the query: "angiogenesis AND homo sapiens AND heart"; and (2) the difference between VEGFB expression in high and low EF patients was the most significant among the pro angiogenic genes *(Table 6).*

### Expression of VEGFB is correlated with outcome after MI

Quantitative PCR was used to confirm microarrays data on VEGFB. Expression values between the 2 groups of 16 MI patients were compared between microarrays and quantitative PCR. Both techniques reported higher levels of VEGFB mRNA expression in high EF patients compared with low EF patients: 1.3 fold (t=3.35; P=0.004) for microarrays, and 1.7 fold (t=3.35; P=0.003) for quantitative PCR (*Figure 8A*). *Figure 8B* displays the significant correlation observed between VEGFB expression and the EF (r=0.39; P=0.03). Therefore, VEGFB expression in blood cells appears to be correlated with outcome after MI.

### Plasma levels of VEGFB are associated with clinical outcome after MI

We then measured VEGFB in the plasma of 140 MI patients, separated in 2 groups, namely those with preserved (LVEF median 57%, range 45-89) and impaired (median 37%, range 17-44) LV function 1 month after MI. Blood sampling was performed the day of MI (n=77), 1 day after MI (n=65) or 2 days after MI (n=12). Plasma VEGFB levels were similar between high and low EF groups at day 0 and day 1 after MI. At day 2 however, VEGFB levels increased in high EF patients (2 fold compared with day 0) whereas they dropped in low EF patients (2.5 fold compared with day 0) (*Figure 9*)*.* These data are in accordance with the up regulation of VEGFB mRNA in high EF patients (*Figure 8*). These results suggest that patients able to increase their production of VEGFB after MI are more prone to have a favourable outcome.

### Independent validation

An independent cohort of 290 MI patients was used to further study the association between VEGFB plasma levels and LV remodelling post MI. Clinical characteristics of this patient population have been published. Mean plasma VEGFB was 64% higher (U Statistic= 8128, P<0.001) in patients in whom ΔEDV (n=138) showed a fall over this period (n=138), compared with patients in whom ΔEDV increased (n=152). These data confirm our observation that VEGFB is associated with LV remodelling after MI.

### Prognostic performance of VEGFB

Our results suggested that VEGFB could represent a potential biomarker of remodelling after MI. Two machine learning models built with several sets of data -obtained either by microarrays, quantitative PCR or plasma determination - were used to test the prognostic performance of VEGFB. Results are shown in *Table* 7. The best performance was achieved when the K* instance based learner was built with VEGFB expression levels measured in blood cells by microarrays from the 32 patients of the test cohort. This model reached a specificity of 75% (12 of 16 low EF patients correctly classified), a sensitivity of 50% (8 of 16 high EF patients correctly classified), and an overall accuracy of 62% (20 of 32 patients correctly classified). AUC was 0.75. When built with plasma VEGFB levels from the validation cohort (290 patients), the maximal prognostic significance provided an AUC of 0.52.

**Table 7. Prediction performances of VEGFB.**

| | | **n** | **Classification model** | **AUC** | **Specificity (%)** | **Sensitivity (%)** | **Accuracy (%)** |
|---|---|---|---|---|---|---|---|
| VEGFB (microarrays) | Day 0 | 32 | **SVM** | 0.56 | 56 | 56 | 56 |
| | | | **K*** | 0.75 | 75 | 50 | 62 |
| VEGFB (PCR) | Day 0 | 32 | **SVM** | 0.68 | 94 | 44 | 69 |
| | | | **K*** | 0.68 | 56 | 56 | 56 |
| VEGFB (plasma) | Day 0 | 77 | **SVM** | 0.5 | 0 | 1 | 66 |
| | | | **K*** | 0.51 | 0 | 92 | 60 |
| | Day 1 | 65 | **SVM** | 0.5 | 0 | 1 | 66 |
| | | | **K*** | 0.47 | 0 | 87 | 57 |
| | Day 2 | 12 | **SVM** | 0.5 | 0 | 1 | 66 |
| | | | **K*** | 0.07 | 0 | 1 | 66 |

| | | | | | | | |
|---|---|---|---|---|---|---|---|
| AUC: Area Under the Receiver Operating Characteristic (ROC) Curve. | | | | | | | |

Specificity indicates the percentage of correctly classified low EF patient; sensitivity indicates the percentage of correctly classified high EF patient; accuracy indicates the percentage of correctly classified high and low EF patients.

## Claims

1. A method of identifying myocardially-infarcted patients having an increased risk of developing heart failure, comprising:
- assaying, post infarction, a body fluid sample from the patient for levels of Vascular Endothelial Growth Factor B (VEGFB), Thrombospondin-1 (THBS1) and Placental Growth Factor (PGF);
- comparing the levels of VEGFB, THBS1 and PGF with a control; and
- determining whether the patient has an increased risk of developing heart failure, based on said comparison.

2. A method according to claim 1, wherein the control is a reference value obtainable from a population of infarcted patients with a range of clinical outcomes, or wherein the control is a corresponding level of VEGFB, THBS1 and PGF from a further sample from said patient.

3. A method according to claim 2, wherein the further sample is an earlier sample from the patient and the following indicates an increased likelihood of said patient suffering from heart failure:
- lower levels of VEGFB in the assayed patient sample compared to the VEGFB earlier sample;
- higher levels of THBS1 in the assayed patient sample compared to the THBS1 earlier sample; and
- higher levels of PGF in the assayed patient sample compared to the PGF earlier sample.

4. A method according to any preceding claims, wherein the body fluid sample taken from the patient is a blood sample, a plasma sample or a serum sample, and/or wherein the levels of VEGFB, THBS1 and PGF assayed are mRNA levels or plasma protein levels, and/or wherein the determination is by nearest neighbor statistical analysis comparison techniques, and/or the method further comprising collecting data on one or more MI patients, including analysis of the levels or values of VEGFB, THBS1 and PGF and the associated clinical outcome for that patient to create feature data for VEGFB, THBS1 and PGF associated with a particular clinical outcome, and/or wherein a classifier is used to determine the prognosis, the classifier including programs such as PAM, Kstar and SVM.

5. A method of identifying myocardially-infarcted (MI) patients having an increased risk of developing heart failure, comprising:
- analyzing a body fluid sample from a post-MI patient for mRNA or plasma protein levels of VEGFB, THBS1 and PGF to determine feature data of the patient, associating this feature data with a particular clinical outcome relating to the incidence of Heart Failure in said patient, and entering this feature data into a database:
- repeating the analysis for a plurality of post-MI patients, to populate the database to contain reference information about the relationship between HF and levels of VEGFB, THBS1 and PGF;
- determining the feature data of a post-MI patient with an unknown prognosis; and
- processing the feature data for the VEGFB, THBS1 and PGF levels from the patient with an unknown prognosis to compare it with the feature data in the database; and
- outputting a likely prognosis for the patient with an unknown prognosis in dependence upon the result of the comparison.

6. A method according to any preceding claims, wherein the determination of an increased risk of heart failure is relative to those infarcted reference samples having comparatively high levels of VEGFB mRNA (log ratio > -1.4), relatively low levels of THBS1 (log ratio < 0) and relatively low levels PGF (log ratio <-0.1), compared to the queried sample, respectively, and preferably wherein ratio values vary by an amount selected from the group consisting of: at least 1%, at least 5%, at least 7%, at least 10%, at least 15%, at least 20%, at least 30%, at least 40%, and up to 50%.

7. A method according to any preceding claims, further comprising assaying the level of BNP and comparing this to a brain natriuretic peptide (BNP) reference sample.

8. A method of establishing a prognosis for a myocardially-infarcted patient for developing heart failure, the method comprising:
- assaying, post infarction, a body fluid sample from the patient for levels of Vascular Endothelial Growth Factor B (VEGFB) and Thrombospondin-1 (THBS1) and placental Growth Factor (PGF); and
- comparing the levels VEGFB, THBS1 and PGF with a control; and
- determining the prognosis for said patient based on said comparison.

9. A method of establishing a prognosis for a myocardially-infarcted patient for developing heart failure, the method comprising:
- assaying, post infarction, a body fluid sample from the patient for levels of Vascular Endothelial Growth Factor B (VEGFB) and Thrombospondin-1 (THBS1) and placental Growth Factor (PGF); and
- questioning a previously built statistical program (also called "classifier") with the levels of VEGFB, THBS1 and PGF;
wherein
- a high level of VEGFB and low levels of THBS1 and PGF are associated with decreased likelihood of developing heart failure; and
- the classifier will dictate whether the patient has a decreased likelihood of developing heart failure.

10. A method of identifying myocardially-infarcted patients having a reduced risk of heart failure, comprising:
- assaying, post-infarction, a body fluid sample from the patient for levels of Vascular Endothelial Growth Factor B (VEGFB) and Thrombospondin-1 (THBS1) and placental Growth Factor (PGF);
- comparing the levels VEGFB, THBS1 and PGF with a control; and
- determining whether the patient has a reduced risk of developing heart failure, based on said comparison.

11. A system comprising a database, the database comprising feature data from MI patient, the data including the clinical outcome of the patient matched to VEGFB, THBS1 and PGF levels assayed post-MI and at least one comparing device to access and/or operate the database and to process the query and adapted to perform the method of any of claims 1-10.

## Patentansprüche

1. Verfahren zum Identifizieren von Myokardinfarkt-Patienten mit einem erhöhten Risiko, Herzversagen zu entwickeln, umfassend:
- Untersuchen nach dem Infarkt einer Körperflüssigkeitsprobe aus dem Patienten auf Spiegel des vaskulären endothelialen Wachstumsfaktors B (VEGFB), von Thrombospondin-1 (THBS1) und Plazenta-Wachstumsfaktor (PGF);
- Vergleichen der VEGFB-, THBS1- und PGF-Spiegel mit einer Kontrolle; und
- Bestimmen, ob der Patient ein erhöhtes Risiko hat, Herzversagen zu entwickeln, auf der Grundlage des Vergleichs.

2. Verfahren nach Anspruch 1, wobei die Kontrolle ein Referenzwert ist, der aus einer Population von Infarktpatienten mit einer Reihe von klinischen Ergebnissen erhalten werden kann, oder wobei die Kontrolle ein entsprechender VEGFB-, THBS1- und PGF-Spiegel aus einer weiteren Probe des Patienten ist.

3. Verfahren nach Anspruch 2, wobei die weitere Probe eine frühere Probe des Patienten ist und Folgendes eine erhöhte Wahrscheinlichkeit des Patienten, an Herzversagen zu leiden, anzeigt:
- niedrigere VEGFB-Spiegel in der untersuchten Patientenprobe im Vergleich zu der früheren VEGFB-Probe;
- höhere THBS1-Spiegel in der untersuchten Patientenprobe im Vergleich zu der früheren THBS1-Probe; und
- höhere PGF-Werte in der untersuchten Patientenprobe im Vergleich zur früheren PGF-Probe.

4. Verfahren nach beliebigen vorhergehenden Ansprüchen, wobei die aus dem Patienten entnommene Körperflüssigkeitsprobe eine Blutprobe, eine Plasmaprobe oder eine Serumprobe ist und/oder wobei die untersuchten VEGFB-, THBS1- und PGF-Spiegel mRNA-Spiegel oder Plasmaproteinspiegel sind und/oder wobei die Bestimmung durch statistische Analyse-Vergleichsverfahren der nächsten Nachbarn erfolgt und/oder wobei das Verfahren zudem das Sammeln von Daten von einem oder mehreren MI-Patienten umfasst, einschließlich der Analyse der Spiegel oder Werte von VEGFB, THBS1 und PGF und dem damit einhergehenden klinischen Ergebnis für diesen Patienten, um Merkmalsdaten für VEGFB, THBS1 und PGF, die mit einem bestimmten klinischen Ergebnis assoziiert sind, zu erzeugen, und/oder wobei ein Klassifikator verwendet wird, um die Prognose zu bestimmen, wobei der Klassifikator Programme wie PAM, Kstar und SVM umfasst.

5. Verfahren zum Identifizieren von Myokardinfarkt-(MI)-Patienten mit einem erhöhten Risiko, Herzversagen zu entwickeln, umfassend:
- Analysieren einer Körperflüssigkeitsprobe von einem Post-MI-Patienten auf mRNA- oder Plasmaproteinspiegel von VEGFB, THBS1 und PGF, um Merkmalsdaten des Patienten zu bestimmen, Assoziieren dieser Merkmalsdaten mit einem bestimmten klinischen Ergebnis in Bezug auf die Inzidenz von Herzversagen in dem Patienten und Eingeben dieser Merkmalsdaten in eine Datenbank;
- Wiederholen der Analyse für eine Vielzahl von Post-MI-Patienten, um die Datenbank so zu füllen, dass Referenzinformationen über die Beziehung zwischen HF und VEGFB-, THBS1- und PGF-Spiegeln aufgenommen werden;
- Bestimmen der Merkmalsdaten eines Post-MI-Patienten mit einer unbekannten Prognose; und
- Verarbeiten der Merkmalsdaten für die VEGFB-, THBS1- und PGF-Spiegel aus dem Patienten mit einer unbekannten Prognose, um sie mit den Merkmalsdaten in der Datenbank zu vergleichen; und
- Ausgeben einer wahrscheinlichen Prognose für den Patienten mit einer unbekannten Prognose in Abhängigkeit vom Ergebnis des Vergleichs.

6. Verfahren nach beliebigen vorhergehenden Ansprüchen, wobei die Bestimmung eines erhöhten Risikos für Herzversagen relativ zu solchen Infarkt-Referenzproben mit vergleichsweise hohen VEGFB-mRNA-Spiegeln (log-Verhältnis > -1,4), relativ niedrigen THBS1-Spiegeln (log-Verhältnis < 0) und relativ niedrigen PGF-Spiegeln (log-Verhältnis < -0,1), verglichen mit der jeweils abgefragten Probe, erfolgt, und wobei die Verhältniswerte vorzugsweise um eine Menge variieren, die ausgewählt ist aus der Gruppe bestehend aus: mindestens 1%, mindestens 5%, mindestens 7%, mindestens 10%, mindestens 15%, mindestens 20%, mindestens 30%, mindestens 40% und bis zu 50%.

7. Verfahren nach beliebigen vorhergehenden Ansprüchen, zudem umfassend das Untersuchen des BNP-Spiegels und das Vergleichen dieses Spiegels mit einer Referenzprobe für natriuretisches Gehirnpeptid (BNP).

8. Verfahren zum Erstellen einer Prognose für einen Myokardinfarkt-Patienten auf die Entwicklung von Herzversagen, wobei das Verfahren umfasst:
- Untersuchen nach dem Infarkt einer Körperflüssigkeitsprobe aus dem Patienten auf die Spiegel des vaskulären endothelialen Wachstumsfaktors B (VEGFB) und von Thrombospondin-1 (THBS1) und Plazenta-Wachstumsfaktor (PGF); und
- Vergleichen der VEGFB-, THBS1- und PGF-Spiegel mit einer Kontrolle; und
- Bestimmen der Prognose für den Patienten auf der Grundlage des Vergleichs.

9. Verfahren zum Erstellen einer Prognose für einen Myokardinfarkt-Patienten auf die Entwicklung von Herzversagen, wobei das Verfahren umfasst:
- Untersuchen nach dem Infarkt einer Körperflüssigkeitsprobe aus dem Patienten auf die Spiegel des vaskulären endothelialen Wachstumsfaktors B (VEGFB) und von Thrombospondin-1 (THBS1) und Plazenta-Wachstumsfaktor (PGF); und
- Abfragen eines zuvor erstellten statistischen Programms (das auch als "Klassifikator" bezeichnet wird) mit den VEGFB-, THBS1- und PGF-Spiegeln;
wobei
- ein hoher VEGFB-Spiegel und niedrige THBS1- und PGF-Spiegel mit einer verminderten Wahrscheinlichkeit für das Entwickeln von Herzversagen verbunden sind; und
- der Klassifikator bestimmt, ob der Patient eine verminderte Wahrscheinlichkeit hat, Herzversagen zu entwickeln.

10. Verfahren zum Identifizieren von Myokardinfarkt-Patienten mit einem verringerten Risiko für Herzversagen, umfassend:
- Untersuchen nach dem Infarkt einer Körperflüssigkeitsprobe aus dem Patienten auf die Spiegel des vaskulären endothelialen Wachstumsfaktors B (VEGFB) und von Thrombospondin-1 (THBS1) und Plazenta-Wachstumsfaktor (PGF); und
- Vergleichen der VEGFB-, THBS1- und PGF-Spiegel mit einer Kontrolle; und
- Bestimmen ob der Patient ein vermindertes Risiko zur Entwicklung von Herzversagen hat, auf der Grundlage des Vergleichs.

11. System, umfassend eine Datenbank, wobei die Datenbank Merkmalsdaten aus dem MI-Patienten umfasst, wobei die Daten das klinische Ergebnis des Patienten beinhalten, das mindestens einem der VEGFB-, THBS1- und PGF-Spiegel entspricht, die nach dem MI getestet wurden, und mindestens eine Vergleichsvorrichtung zum Zugreifen auf die und/oder Betreiben der Datenbank und zum Verarbeiten der Abfrage, und die zur Ausführung des Verfahrens nach einem der Ansprüche 1 bis 10 ausgelegt ist.

## Revendications

1. Procédé d'identification de patients atteints d'infarctus du myocarde ayant un risque accru de développer une insuffisance cardiaque, comprenant :
- le dosage, post-infarctus, d'un échantillon de fluide corporel du patient pour les taux de facteur de croissance endothélial vasculaire B (VEGFB), de thrombospondine 1 (THBS1) et de facteur de croissance placentaire (PGF) ;
- la comparaison des taux de VEGFB, THBS1 et PGF à un témoin ; et
- la détermination que le patient présente ou non un risque accru de développer une insuffisance cardiaque, sur la base de ladite comparaison.

2. Procédé selon la revendication 1, dans lequel le démon est une valeur de référence pouvant être obtenue à partir d'une population de patients atteints d'infarctus présentant une plage de résultats cliniques, ou dans lequel le témoin est un taux correspondant de VEGFB, THBS1 et PGF d'un autre échantillon dudit patient.

3. Procédé selon la revendication 2, dans lequel votre échantillon est un échantillon antérieur du patient et les éléments suivants indiquent une probabilité accrue que ledit patient souffre d'insuffisance cardiaque :
- des taux plus faibles de VEGFB dans l'échantillon de patient analysé par rapport à l'échantillon antérieur de VEGFB ;
- des taux plus élevés de THBS1 dans l'échantillon de patient analysé par rapport à l'échantillon antérieur de THBS1 ; et
- des taux plus élevés de PGF dans l'échantillon de patient analysé par rapport à l'échantillon antérieur de PGF.

4. Procédé selon l'une quelconque des revendications précédentes, dans lequel l'échantillon de fluide corporel prélevé à partir du patient est un échantillon de sang, un échantillon de plasma ou un échantillon de sérum, et/ou dans lequel les taux de VEGFB, THBS1 et PGF dosés sont des taux d'ARNm ou des taux de protéine plasmatique, et/ou dans lequel la détermination est effectuée par des techniques de comparaison d'analyse statistique des plus proches voisins, et/ou le procédé comprenant en outre la collecte de données sur un ou plusieurs patients IM, comprenant l'analyse des taux ou valeurs de VEGFB, THBS1 et PGF et du résultat cliniques associé pour ce patient afin de créer des données caractéristiques pour VEGFB, THBS1 et PGF associées à un résultat cliniques particulier, et/ou dans lequel un classificateur est utilisé pour déterminer le pronostic, le classificateur comprenant des programmes tels que PAM, Kstar et SVM.

5. Procédé d'identification de patients atteints d'infarctus du myocarde ayant un risque accru de développer une insuffisance cardiaque, comprenant :
- l'analyse d'un échantillon de fluide corporel d'un patient post-IM pour les taux d'ARNm ou de protéine plasmatique de VEGFB, THBS1 et PGF pour déterminer des données caractéristiques du patient, l'association de ces données caractéristiques à un résultat cliniques particulier lié à l'incidence d'insuffisance cardiaque chez ledit patient, et l'entrée de ces données caractéristiques dans une base de données ;
- la répétition de l'analyse pour une pluralité de patients post-IM, pour peupler la base de données de manière à contenir des informations de référence sur la relation entre l'insuffisance cardiaque et les taux de VEGFB, THBS1 et PGF ;
- la détermination des données caractéristiques d'un patient post-IM ayant un pronostic inconnu ; et
- le traitement des données caractéristiques pour les taux de VEGFB, THBS1 et PGF du patient ayant un pronostic inconnu afin de comparer celles-ci aux données caractéristiques dans la base de données ; et
- la délivrance en sortie d'un pronostic probable pour le patient ayant un pronostic inconnu en fonction du résultat de la comparaison.

6. Procédé selon l'une quelconque des revendications précédentes, dans lequel la détermination d'un risque accru d'insuffisance cardiaque est effectuée par rapport aux échantillons de référence avec infarctus ayant des taux comparativement élevés d'ARNm de VEGFB (rapport logarithmique > -1,4), des taux relativement faibles de THBS1 (rapport logarithmique < 0) et des taux relativement faibles de PGF (rapport logarithmique < -0,1), par rapport à l'échantillon examiné, respectivement, et, de préférence, dans lequel les valeurs de rapports varient d'une quantité choisie dans le groupe constitué de : au moins 1 %, au moins 5 %, au moins 7 %, au moins 10 %, au moins 15 %, au moins 20 %, au moins 30 %, au moins 40 %, et jusqu'à 50 %.

7. Procédé selon l'une quelconque des revendications précédentes, comprenant en outre le dosage du taux de BNP et la comparaison de celui-ci à un échantillon de référence de peptide cérébral natriurétique (BNP).

8. Procédé d'établissement d'un pronostic pour un patient atteint d'infarctus du myocarde du développement d'une insuffisance cardiaque, le procédé comprenant :
- le dosage, post-infarctus, d'un échantillon de fluide corporel du patient pour les taux de facteur de croissance endothélial vasculaire B (VEGFB), de thrombospondine 1 (THBS1) et de facteur de croissance placentaire (PGF) ; et
- la comparaison des taux de VEGFB, THBS1 et PGF à un témoin ; et
- la détermination du pronostic pour ledit patient sur la base de ladite comparaison.

9. Procédé d'établissement d'un pronostic pour un patient atteint d'infarctus du myocarde du développement d'une insuffisance cardiaque, le procédé comprenant :
- le dosage, post-infarctus, d'un échantillon de fluide corporel du patient pour les taux de facteur de croissance endothélial vasculaire B (VEGFB), de thrombospondine 1 (THBS1) et de facteur de croissance placentaire (PGF) ; et
- l'interrogation d'un programme statistique précédemment développé (également appelé "classificateur") avec les taux de VEGFB, THBS1 et PGF ;
dans lequel
- un taux élevé de VEGFB et des taux faibles de THBS1 et PGF sont associés à une probabilité réduite de développer une insuffisance cardiaque ; et
- le classificateur détermine si le patient présent une probabilité réduite de développer une insuffisance cardiaque.

10. Procédé d'identification de patients atteints d'infarctus du myocarde présentant un risque réduit d'insuffisance cardiaque, comprenant :
- le dosage, post-infarctus, d'un échantillon de fluide corporel du patient pour les taux de facteur de croissance endothélial vasculaire B (VEGFB), de thrombospondine 1 (THBS1) et de facteur de croissance placentaire (PGF) ;
- la comparaison des taux de VEGFB, THBS1 et PGF à un témoin ; et
- la détermination que le patient présente ou non un risque réduit de développer une insuffisance cardiaque, sur la base de ladite comparaison.

11. Système comprenant une base de données, la base de données comprenant des données caractéristiques de patient IM, les données comprenant le résultat clinique du patient mis en correspondance avec les taux de VEGFB, THBS1 et PGF dosés post-IM et au moins un dispositif de comparaison pour accéder à et/ou manipuler la base de données et pour traiter l'interrogation et adapté pour conduire le procédé de l'une quelconque des revendications 1 à 10.


### SEQUENCE LISTING

<110> Centre de Recherche Public de la Santé
<120> BIOMARKERS
<130> 08171-2.WO
<160> 13
<170> PatentIn version 3.3
<210> 1
   <211> 1172
   <212> DNA
   <213> Homo sapiens
<400> 1
   gcgatgcggg cgcccccggc gggcggcccc ggcgggcacc atgagccctc tgctccgccg      60
   cctgctgctc gccgcactcc tgcagctggc ccccgcccag gcccctgtct cccagcctga    120
   tgcccctggc caccagagga aagtggtgtc atggatagat gtgtatactc gcgctacctg    180
   ccagccccgg gaggtggtgg tgcccttgac tgtggagctc atgggcaccg tggccaaaca    240
   gctggtgccc agctgcgtga ctgtgcagcg ctgtggtggc tgctgccctg acgatggcct    300
   ggagtgtgtg cccactgggc agcaccaagt ccggatgcag atcctcatga tccggtaccc    360
   gagcagtcag ctgggggaga tgtccctgga agaacacagc cagtgtgaat gcagacctaa    420
   aaaaaaggac agtgctgtga agccagacag ggctgccact ccccaccacc gtccccagcc    480
   ccgttctgtt ccgggctggg actctgcccc cggagcaccc tccccagctg acatcaccca    540
   tcccactcca gccccaggcc cctctgccca cgctgcaccc agcaccacca gcgccctgac    600
   ccccggacct gccgctgccg ctgccgacgc cgcagcttcc tccgttgcca agggcggggc    660
   ttagagctca acccagacac ctgcaggtgc cggaagctgc gaaggtgaca catggctttt    720
   cagactcagc agggtgactt gcctcagagg ctatatccca gtgggggaac aaagaggagc    780
   ctggtaaaaa acagccaagc ccccaagacc tcagcccagg cagaagctgc tctaggacct    840
   gggcctctca gagggctctt ctgccatccc ttgtctccct gaggccatca tcaaacagga    900
   cagagttgga agaggagact gggaggcagc aagaggggtc acataccagc tcaggggaga    960
   atggagtact gtctcagttt ctaaccactc tgtgcaagta agcatcttac aactggctct   1020
   tcctcccctc actaagaaga cccaaacctc tgcataatgg gatttgggct ttggtacaag   1080
   aactgtgacc cccaaccctg ataaaagaga tggaaggaaa aaaaaaaaaa aaaaaaaaaa   1140
   aaaaaaaaaa aaaaaaaaaa aaaaaaaaaa aa                                 1172
<210> 2
   <211> 624
   <212> DNA
   <213> Homo sapiens
<400> 2
   atgagccctc tgctccgccg cctgctgctc gccgcactcc tgcagctggc ccccgcccag     60
   gcccctgtct cccagcctga tgcccctggc caccagagga aagtggtgtc atggatagat    120
   gtgtatactc gcgctacctg ccagccccgg gaggtggtgg tgcccttgac tgtggagctc    180
   atgggcaccg tggccaaaca gctggtgccc agctgcgtga ctgtgcagcg ctgtggtggc    240
   tgctgccctg acgatggcct ggagtgtgtg cccactgggc agcaccaagt ccggatgcag    300
   atcctcatga tccggtaccc gagcagtcag ctgggggaga tgtccctgga agaacacagc    360
   cagtgtgaat gcagacctaa aaaaaaggac agtgctgtga agccagacag ggctgccact    420
   ccccaccacc gtccccagcc ccgttctgtt ccgggctggg actctgcccc cggagcaccc    480
   tccccagctg acatcaccca tcccactcca gccccaggcc cctctgccca cgctgcaccc    540
   agcaccacca gcgccctgac ccccggacct gccgctgccg ctgccgacgc cgcagcttcc    600
   tccgttgcca agggcggggc ttag                                           624
<210> 3
   <211> 207
   <212> PRT
   <213> Homo sapiens
<400> 3
   Met Ser Pro Leu Leu Arg Arg Leu Leu Leu Ala Ala Leu Leu Gln Leu
   1               5                   10                  15
   Ala Pro Ala Gln Ala Pro Val Ser Gln Pro Asp Ala Pro Gly His Gln
               20                  25                  30
   Arg Lys Val Val Ser Trp Ile Asp Val Tyr Thr Arg Ala Thr Cys Gln
           35                  40                  45
   Pro Arg Glu Val Val Val Pro Leu Thr Val Glu Leu Met Gly Thr Val
       50                  55                  60
   Ala Lys Gln Leu Val Pro Ser Cys Val Thr Val Gln Arg Cys Gly Gly
   65                  70                  75                  80
   Cys Cys Pro Asp Asp Gly Leu Glu Cys Val Pro Thr Gly Gln His Gln
                   85                  90                  95
   Val Arg Met Gln Ile Leu Met Ile Arg Tyr Pro Ser Ser Gln Leu Gly
               100                 105                 110
   Glu Met Ser Leu Glu Glu His Ser Gln Cys Glu Cys Arg Pro Lys Lys
           115                 120                 125
   Lys Asp Ser Ala Val Lys Pro Asp Arg Ala Ala Thr Pro His His Arg
       130                 135                 140
   Pro Gln Pro Arg Ser Val Pro Gly Trp Asp Ser Ala Pro Gly Ala Pro
   145                 150                 155                 160
   Ser Pro Ala Asp Ile Thr His Pro Thr Pro Ala Pro Gly Pro Ser Ala
                   165                 170                 175
   His Ala Ala Pro Ser Thr Thr Ser Ala Leu Thr Pro Gly Pro Ala Ala
               180                 185                 190
   Ala Ala Ala Asp Ala Ala Ala Ser Ser Val Ala Lys Gly Gly Ala
           195                 200                 205
<210> 4
   <211> 5820
   <212> DNA
   <213> Homo sapiens
<400> 4
   agccgctgcg cccgagctgg cctgcgagtt cagggctcct gtcgctctcc aggagcaacc     60
   tctactccgg acgcacaggc attccccgcg cccctccagc cctcgccgcc ctcgccaccg    120
   ctcccggccg ccgcgctccg gtacacacag gatccctgct gggcaccaac agctccacca    180
   tggggctggc ctggggacta ggcgtcctgt tcctgatgca tgtgtgtggc accaaccgca    240
   ttccagagtc tggcggagac aacagcgtgt ttgacatctt tgaactcacc ggggccgccc    300
   gcaaggggtc tgggcgccga ctggtgaagg gccccgaccc ttccagccca gctttccgca    360
   tcgaggatgc caacctgatc ccccctgtgc ctgatgacaa gttccaagac ctggtggatg    420
   ctgtgcgggc agaaaagggt ttcctccttc tggcatccct gaggcagatg aagaagaccc    480
   ggggcacgct gctggccctg gagcggaaag accactctgg ccaggtcttc agcgtggtgt    540
   ccaatggcaa ggcgggcacc ctggacctca gcctgaccgt ccaaggaaag cagcacgtgg    600
   tgtctgtgga agaagctctc ctggcaaccg gccagtggaa gagcatcacc ctgtttgtgc    660
   aggaagacag ggcccagctg tacatcgact gtgaaaagat ggagaatgct gagttggacg    720
   tccccatcca aagcgtcttc accagagacc tggccagcat cgccagactc cgcatcgcaa    780
   aggggggcgt caatgacaat ttccaggggg tgctgcagaa tgtgaggttt gtctttggaa    840
   ccacaccaga agacatcctc aggaacaaag gctgctccag ctctaccagt gtcctcctca    900
   cccttgacaa caacgtggtg aatggttcca gccctgccat ccgcactaac tacattggcc    960
   acaagacaaa ggacttgcaa gccatctgcg gcatctcctg tgatgagctg tccagcatgg   1020
   tcctggaact caggggcctg cgcaccattg tgaccacgct gcaggacagc atccgcaaag   1080
   tgactgaaga gaacaaagag ttggccaatg agctgaggcg gcctccccta tgctatcaca   1140
   acggagttca gtacagaaat aacgaggaat ggactgttga tagctgcact gagtgtcact   1200
   gtcagaactc agttaccatc tgcaaaaagg tgtcctgccc catcatgccc tgctccaatg   1260
   ccacagttcc tgatggagaa tgctgtcctc gctgttggcc cagcgactct gcggacgatg   1320
   gctggtctcc atggtccgag tggacctcct gttctacgag ctgtggcaat ggaattcagc   1380
   agcgcggccg ctcctgcgat agcctcaaca accgatgtga gggctcctcg gtccagacac   1440
   ggacctgcca cattcaggag tgtgacaaga gatttaaaca ggatggtggc tggagccact   1500
   ggtccccgtg gtcatcttgt tctgtgacat gtggtgatgg tgtgatcaca aggatccggc   1560
   tctgcaactc tcccagcccc cagatgaacg ggaaaccctg tgaaggcgaa gcgcgggaga   1620
   ccaaagcctg caagaaagac gcctgcccca tcaatggagg ctggggtcct tggtcaccat   1680
   gggacatctg ttctgtcacc tgtggaggag gggtacagaa acgtagtcgt ctctgcaaca   1740
   accccacacc ccagtttgga ggcaaggact gcgttggtga tgtaacagaa aaccagatct   1800
   gcaacaagca ggactgtcca attgatggat gcctgtccaa tccctgcttt gccggcgtga   1860
   agtgtactag ctaccctgat ggcagctgga aatgtggtgc ttgtccccct ggttacagtg   1920
   gaaatggcat ccagtgcaca gatgttgatg agtgcaaaga agtgcctgat gcctgcttca   1980
   accacaatgg agagcaccgg tgtgagaaca cggaccccgg ctacaactgc ctgccctgcc   2040
   ccccacgctt caccggctca cagcccttcg gccagggtgt cgaacatgcc acggccaaca   2100
   aacaggtgtg caagccccgt aacccctgca cggatgggac ccacgactgc aacaagaacg   2160
   ccaagtgcaa ctacctgggc cactatagcg accccatgta ccgctgcgag tgcaagcctg   2220
   gctacgctgg caatggcatc atctgcgggg aggacacaga cctggatggc tggcccaatg   2280
   agaacctggt gtgcgtggcc aatgcgactt accactgcaa aaaggataat tgccccaacc   2340
   ttcccaactc agggcaggaa gactatgaca aggatggaat tggtgatgcc tgtgatgatg   2400
   acgatgacaa tgataaaatt ccagatgaca gggacaactg tccattccat tacaacccag   2460
   ctcagtatga ctatgacaga gatgatgtgg gagaccgctg tgacaactgt ccctacaacc   2520
   acaacccaga tcaggcagac acagacaaca atggggaagg agacgcctgt gctgcagaca   2580
   ttgatggaga cggtatcctc aatgaacggg acaactgcca gtacgtctac aatgtggacc   2640
   agagagacac tgatatggat ggggttggag atcagtgtga caattgcccc ttggaacaca   2700
   atccggatca gctggactct gactcagacc gcattggaga tacctgtgac aacaatcagg   2760
   atattgatga agatggccac cagaacaatc tggacaactg tccctatgtg cccaatgcca   2820
   accaggctga ccatgacaaa gatggcaagg gagatgcctg tgaccacgat gatgacaacg   2880
   atggcattcc tgatgacaag gacaactgca gactcgtgcc caatcccgac cagaaggact   2940
   ctgacggcga tggtcgaggt gatgcctgca aagatgattt tgaccatgac agtgtgccag   3000
   acatcgatga catctgtcct gagaatgttg acatcagtga gaccgatttc cgccgattcc 3060
   agatgattcc tctggacccc aaagggacat cccaaaatga ccctaactgg gttgtacgcc 3120
   atcagggtaa agaactcgtc cagactgtca actgtgatcc tggactcgct gtaggttatg 3180
   atgagtttaa tgctgtggac ttcagtggca ccttcttcat caacaccgaa agggacgatg 3240
   actatgctgg atttgtcttt ggctaccagt ccagcagccg cttttatgtt gtgatgtgga 3300
   agcaagtcac ccagtcctac tgggacacca accccacgag ggctcaggga tactcgggcc 3360
   tttctgtgaa agttgtaaac tccaccacag ggcctggcga gcacctgcgg aacgccctgt 3420
   ggcacacagg aaacacccct ggccaggtgc gcaccctgtg gcatgaccct cgtcacatag 3480
   gctggaaaga tttcaccgcc tacagatggc gtctcagcca caggccaaag acgggtttca 3540
   ttagagtggt gatgtatgaa gggaagaaaa tcatggctga ctcaggaccc atctatgata 3600
   aaacctatgc tggtggtaga ctagggttgt ttgtcttctc tcaagaaatg gtgttcttct 3660
   ctgacctgaa atacgaatgt agagatccct aatcatcaaa ttgttgattg aaagactgat 3720
   cataaaccaa tgctggtatt gcaccttctg gaactatggg cttgagaaaa cccccaggat 3780
   cacttctcct tggcttcctt cttttctgtg cttgcatcag tgtggactcc tagaacgtgc 3840
   gacctgcctc aagaaaatgc agttttcaaa aacagactca gcattcagcc tccaatgaat 3900
   aagacatctt ccaagcatat aaacaattgc tttggtttcc ttttgaaaaa gcatctactt 3960
   gcttcagttg ggaaggtgcc cattccactc tgcctttgtc acagagcagg gtgctattgt 4020
   gaggccatct ctgagcagtg gactcaaaag cattttcagg catgtcagag aagggaggac 4080
   tcactagaat tagcaaacaa aaccaccctg acatcctcct tcaggaacac ggggagcaga 4140
   ggccaaagca ctaaggggag ggcgcatacc cgagacgatt gtatgaagaa aatatggagg 4200
   aactgttaca tgttcggtac taagtcattt tcaggggatt gaaagactat tgctggattt 4260
   catgatgctg actggcgtta gctgattaac ccatgtaaat aggcacttaa atagaagcag 4320
   gaaagggaga caaagactgg cttctggact tcctccctga tccccaccct tactcatcac 4380
   ctgcagtggc cagaattagg gaatcagaat caaaccagtg taaggcagtg ctggctgcca 4440
   ttgcctggtc acattgaaat tggtggcttc attctagatg tagcttgtgc agatgtagca 4500
   ggaaaatagg aaaacctacc atctcagtga gcaccagctg cctcccaaag gaggggcagc 4560
   cgtgcttata tttttatggt tacaatggca caaaattatt atcaacctaa ctaaaacatt 4620
   ccttttctct tttttcctga attatcatgg agttttctaa ttctctcttt tggaatgtag 4680
   atttttttta aatgctttac gatgtaaaat atttattttt tacttattct ggaagatctg 4740
   gctgaaggat tattcatgga acaggaagaa gcgtaaagac tatccatgtc atctttgttg 4800
   agagtcttcg tgactgtaag attgtaaata cagattattt attaactctg ttctgcctgg 4860
   aaatttaggc ttcatacgga aagtgtttga gagcaagtag ttgacattta tcagcaaatc   4920
   tcttgcaaga acagcacaag gaaaatcagt ctaataagct gctctgcccc ttgtgctcag   4980
   agtggatgtt atgggattct ttttttctct gttttatctt ttcaagtgga attagttggt   5040
   tatccatttg caaatgtttt aaattgcaaa gaaagccatg aggtcttcaa tactgtttta   5100
   ccccatccct tgtgcatatt tccagggaga aggaaagcat atacactttt ttctttcatt   5160
   tttccaaaag agaaaaaaat gacaaaaggt gaaacttaca tacaaatatt acctcatttg   5220
   ttgtgtgact gagtaaagaa tttttggatc aagcggaaag agtttaagtg tctaacaaac   5280
   ttaaagctac tgtagtacct aaaaagtcag tgttgtacat agcataaaaa ctctgcagag   5340
   aagtattccc aataaggaaa tagcattgaa atgttaaata caatttctga aagttatgtt   5400
   ttttttctat catctggtat accattgctt tatttttata aattattttc tcattgccat   5460
   tggaatagat atctcagatt gtgtagatat gctatttaaa taatttatca ggaaatactg   5520
   cctgtagagt tagtatttct atttttatat aatgtttgca cactgaattg aagaattgtt   5580
   ggttttttct tttttttgtt ttgttttttt tttttttttt ttttgctttt gacctcccat   5640
   ttttactatt tgccaatacc tttttctagg aatgtgcttt tttttgtaca catttttatc   5700
   cattttacat tctaaagcag tgtaagttgt atattactgt ttcttatgta caaggaacaa   5760
   caataaatca tatggaaatt tatatttata aaaaaaaaaa aaaaaaaaaa aaaaaaaaaa   5820
<210> 5
   <211> 3513
   <212> DNA
   <213> Homo sapiens
<400> 5
   atggggctgg cctggggact aggcgtcctg ttcctgatgc atgtgtgtgg caccaaccgc     60
   attccagagt ctggcggaga caacagcgtg tttgacatct ttgaactcac cggggccgcc    120
   cgcaaggggt ctgggcgccg actggtgaag ggccccgacc cttccagccc agctttccgc    180
   atcgaggatg ccaacctgat cccccctgtg cctgatgaca agttccaaga cctggtggat     240
   gctgtgcggg cagaaaaggg tttcctcctt ctggcatccc tgaggcagat gaagaagacc    300
   cggggcacgc tgctggccct ggagcggaaa gaccactctg gccaggtctt cagcgtggtg    360
   tccaatggca aggcgggcac cctggacctc agcctgaccg tccaaggaaa gcagcacgtg    420
   gtgtctgtgg aagaagctct cctggcaacc ggccagtgga agagcatcac cctgtttgtg    480
   caggaagaca gggcccagct gtacatcgac tgtgaaaaga tggagaatgc tgagttggac    540
   gtccccatcc aaagcgtctt caccagagac ctggccagca tcgccagact ccgcatcgca    600
   aaggggggcg tcaatgacaa tttccagggg gtgctgcaga atgtgaggtt tgtctttgga    660
   accacaccag aagacatcct caggaacaaa ggctgctcca gctctaccag tgtcctcctc    720
   acccttgaca acaacgtggt gaatggttcc agccctgcca tccgcactaa ctacattggc    780
   cacaagacaa aggacttgca agccatctgc ggcatctcct gtgatgagct gtccagcatg    840
   gtcctggaac tcaggggcct gcgcaccatt gtgaccacgc tgcaggacag catccgcaaa    900
   gtgactgaag agaacaaaga gttggccaat gagctgaggc ggcctcccct atgctatcac    960
   aacggagttc agtacagaaa taacgaggaa tggactgttg atagctgcac tgagtgtcac   1020
   tgtcagaact cagttaccat ctgcaaaaag gtgtcctgcc ccatcatgcc ctgctccaat   1080
   gccacagttc ctgatggaga atgctgtcct cgctgttggc ccagcgactc tgcggacgat   1140
   ggctggtctc catggtccga gtggacctcc tgttctacga gctgtggcaa tggaattcag   1200
   cagcgcggcc gctcctgcga tagcctcaac aaccgatgtg agggctcctc ggtccagaca   1260
   cggacctgcc acattcagga gtgtgacaag agatttaaac aggatggtgg ctggagccac   1320
   tggtccccgt ggtcatcttg ttctgtgaca tgtggtgatg gtgtgatcac aaggatccgg   1380
   ctctgcaact ctcccagccc ccagatgaac gggaaaccct gtgaaggcga agcgcgggag   1440
   accaaagcct gcaagaaaga cgcctgcccc atcaatggag gctggggtcc ttggtcacca   1500
   tgggacatct gttctgtcac ctgtggagga ggggtacaga aacgtagtcg tctctgcaac   1560
   aaccccacac cccagtttgg aggcaaggac tgcgttggtg atgtaacaga aaaccagatc   1620
   tgcaacaagc aggactgtcc aattgatgga tgcctgtcca atccctgctt tgccggcgtg   1680
   aagtgtacta gctaccctga tggcagctgg aaatgtggtg cttgtccccc tggttacagt   1740
   ggaaatggca tccagtgcac agatgttgat gagtgcaaag aagtgcctga tgcctgcttc   1800
   aaccacaatg gagagcaccg gtgtgagaac acggaccccg gctacaactg cctgccctgc   1860
   cccccacgct tcaccggctc acagcccttc ggccagggtg tcgaacatgc cacggccaac   1920
   aaacaggtgt gcaagccccg taacccctgc acggatggga cccacgactg caacaagaac   1980
   gccaagtgca actacctggg ccactatagc gaccccatgt accgctgcga gtgcaagcct   2040
   ggctacgctg gcaatggcat catctgcggg gaggacacag acctggatgg ctggcccaat   2100
   gagaacctgg tgtgcgtggc caatgcgact taccactgca aaaaggataa ttgccccaac   2160
   cttcccaact cagggcagga agactatgac aaggatggaa ttggtgatgc ctgtgatgat   2220
   gacgatgaca atgataaaat tccagatgac agggacaact gtccattcca ttacaaccca   2280
   gctcagtatg actatgacag agatgatgtg ggagaccgct gtgacaactg tccctacaac   2340
   cacaacccag atcaggcaga cacagacaac aatggggaag gagacgcctg tgctgcagac   2400
   attgatggag acggtatcct caatgaacgg gacaactgcc agtacgtcta caatgtggac   2460
   cagagagaca ctgatatgga tggggttgga gatcagtgtg acaattgccc cttggaacac   2520
   aatccggatc agctggactc tgactcagac cgcattggag atacctgtga caacaatcag   2580
   gatattgatg aagatggcca ccagaacaat ctggacaact gtccctatgt gcccaatgcc   2640
   aaccaggctg accatgacaa agatggcaag ggagatgcct gtgaccacga tgatgacaac   2700
   gatggcattc ctgatgacaa ggacaactgc agactcgtgc ccaatcccga ccagaaggac   2760
   tctgacggcg atggtcgagg tgatgcctgc aaagatgatt ttgaccatga cagtgtgcca   2820
   gacatcgatg acatctgtcc tgagaatgtt gacatcagtg agaccgattt ccgccgattc   2880
   cagatgattc ctctggaccc caaagggaca tcccaaaatg accctaactg ggttgtacgc   2940
   catcagggta aagaactcgt ccagactgtc aactgtgatc ctggactcgc tgtaggttat   3000
   gatgagttta atgctgtgga cttcagtggc accttcttca tcaacaccga aagggacgat   3060
   gactatgctg gatttgtctt tggctaccag tccagcagcc gcttttatgt tgtgatgtgg   3120
   aagcaagtca cccagtccta ctgggacacc aaccccacga gggctcaggg atactcgggc   3180
   ctttctgtga aagttgtaaa ctccaccaca gggcctggcg agcacctgcg gaacgccctg   3240
   tggcacacag gaaacacccc tggccaggtg cgcaccctgt ggcatgaccc tcgtcacata   3300
   ggctggaaag atttcaccgc ctacagatgg cgtctcagcc acaggccaaa gacgggtttc   3360
   attagagtgg tgatgtatga agggaagaaa atcatggctg actcaggacc catctatgat   3420
   aaaacctatg ctggtggtag actagggttg tttgtcttct ctcaagaaat ggtgttcttc   3480
   tctgacctga aatacgaatg tagagatccc taa                                3513
<210> 6
   <211> 1170
   <212> PRT
   <213> Homo sapiens
<400> 6
<210> 7
   <211> 1758
   <212> DNA
   <213> Homo sapiens
<400> 7
<210> 8
   <211> 513
   <212> DNA
   <213> Homo sapiens
<400> 8
   atgccggtca tgaggctgtt cccttgcttc ctgcagctcc tggccgggct ggcgctgcct     60
   gctgtgcccc cccagcagtg ggccttgtct gctgggaacg gctcgtcaga ggtggaagtg    120
   gtacccttcc aggaagtgtg gggccgcagc tactgccggg cgctggagag gctggtggac    180
   gtcgtgtccg agtaccccag cgaggtggag cacatgttca gcccatcctg tgtctccctg    240
   ctgcgctgca ccggctgctg cggcgatgag aatctgcact gtgtgccggt ggagacggcc    300
   aatgtcacca tgcagctcct aaagatccgt tctggggacc ggccctccta cgtggagctg    360
   acgttctctc agcacgttcg ctgcgaatgc cggcctctgc gggagaagat gaagccggaa    420
   aggaggagac ccaagggcag gggaagaggg aggagagaga agcagagacc cacagactgc    480
   cacctgtgcg gcgatgctgt tccccggagg taa                                 513
<210> 9
   <211> 170
   <212> PRT
   <213> Homo sapiens
<400> 9
   Met Pro Val Met Arg Leu Phe Pro Cys Phe Leu Gln Leu Leu Ala Gly
   1               5                   10                  15
   Leu Ala Leu Pro Ala Val Pro Pro Gln Gln Trp Ala Leu Ser Ala Gly
               20                  25                  30
   Asn Gly Ser Ser Glu Val Glu Val Val Pro Phe Gln Glu Val Trp Gly
           35                  40                  45
   Arg Ser Tyr Cys Arg Ala Leu Glu Arg Leu Val Asp Val Val Ser Glu
       50                  55                  60
   Tyr Pro Ser Glu Val Glu His Met Phe Ser Pro Ser Cys Val Ser Leu
   65                  70                  75                  80
   Leu Arg Cys Thr Gly Cys Cys Gly Asp Glu Asn Leu His Cys Val Pro
                   85                  90                  95
   Val Glu Thr Ala Asn Val Thr Met Gln Leu Leu Lys Ile Arg Ser Gly
               100                 105                 110
   Asp Arg Pro Ser Tyr Val Glu Leu Thr Phe Ser Gln His Val Arg Cys
           115                 120                 125
   Glu Cys Arg Pro Leu Arg Glu Lys Met Lys Pro Glu Arg Arg Arg Pro
       130                 135                 140
   Lys Gly Arg Gly Lys Arg Arg Arg Glu Lys Gln Arg Pro Thr Asp Cys
   145                 150                 155                 160
   His Leu Cys Gly Asp Ala Val Pro Arg Arg
                   165                 170
<210> 10
   <211> 708
   <212> DNA
   <213> Homo sapiens
<400> 10
   ccccgcaggc tgagggcagg tgggaagcaa acccggacgc atcgcagcag cagcagcagc      60
   agcagaagca gcagcagcag cctccgcagt ccctccagag acatggatcc ccagacagca     120
   ccttcccggg cgctcctgct cctgctcttc ttgcatctgg ctttcctggg aggtcgttcc     180
   cacccgctgg gcagccccgg ttcagcctcg gacttggaaa cgtccgggtt acaggagcag     240
   cgcaaccatt tgcagggcaa actgtcggag ctgcaggtgg agcagacatc cctggagccc     300
   ctccaggaga gcccccgtcc cacaggtgtc tggaagtccc gggaggtagc caccgagggc     360
   atccgtgggc accgcaaaat ggtcctctac accctgcggg caccacgaag ccccaagatg     420
   gtgcaagggt ctggctgctt tgggaggaag atggaccgga tcagctcctc cagtggcctg     480
   ggctgcaaag tgctgaggcg gcattaagag gaagtcctgg ctgcagacac ctgcttctga     540
   ttccacaagg ggctttttcc tcaaccctgt ggccgccttt gaagtgactc atttttttaa     600
   tgtatttatg tatttatttg attgttttat ataagatggt ttcttacctt tgagcacaaa 660
   atttccacgg tgaaataaag tcaacattat aagctttaaa aaaaaaaa 708
<210> 11
   <211> 405
   <212> DNA
   <213> Homo sapiens
<400> 11
   atggatcccc agacagcacc ttcccgggcg ctcctgctcc tgctcttctt gcatctggct 60
   ttcctgggag gtcgttccca cccgctgggc agccccggtt cagcctcgga cttggaaacg 120
   tccgggttac aggagcagcg caaccatttg cagggcaaac tgtcggagct gcaggtggag 180
   cagacatccc tggagcccct ccaggagagc cccgtcccca caggtgtctg gaagtcccgg 240
   gaggtagcca ccgagggcat ccgtgggcac cgcaaaatgg tcctctacac cctgcgggca 300
   ccacgaagcc ccaagatggt gcaagggtct ggctgctttg ggaggaagat ggaccggatc 360
   agctcctcca gtggcctggg ctgcaaagtg ctgaggcggc attaa 405
<210> 12
   <211> 134
   <212> PRT
   <213> Homo sapiens
<400> 12
   Met Asp Pro Gln Thr Ala Pro Ser Arg Ala Leu Leu Leu Leu Leu Phe
   1               5                   10                  15
   Leu His Leu Ala Phe Leu Gly Gly Arg Ser His Pro Leu Gly Ser Pro
               20                  25                  30
   Gly Ser Ala Ser Asp Leu Glu Thr Ser Gly Leu Gln Glu Gln Arg Asn
           35                  40                  45
   His Leu Gln Gly Lys Leu Ser Glu Leu Gln Val Glu Gln Thr Ser Leu
       50                  55                  60
   Glu Pro Leu Gln Glu Ser Pro Arg Pro Thr Gly Val Trp Lys Ser Arg
   65                  70                  75                  80
   Glu Val Ala Thr Glu Gly Ile Arg Gly His Arg Lys Met Val Leu Tyr
                   85                  90                  95
   Thr Leu Arg Ala Pro Arg Ser Pro Lys Met Val Gln Gly Ser Gly Cys
               100                 105                 110
   Phe Gly Arg Lys Met Asp Arg Ile Ser Ser Ser Ser Gly Leu Gly Cys
           115                 120                 125
   Lys Val Leu Arg Arg His
       130
<210> 13
   <211> 184
   <212> PRT
   <213> Homo sapiens
<400> 13
   Phe Asp Ala Ile Ala Glu Ile Gly Asn Gln Leu Tyr Leu Phe Lys Asp
   1               5                   10                  15
   Gly Lys Tyr Trp Arg Phe Ser Glu Gly Arg Gly Ser Arg Pro Gln Gly
               20                  25                  30
   Pro Phe Leu Ile Ala Asp Lys Trp Pro Ala Leu Pro Arg Lys Leu Asp
           35                  40                  45
   Ser Val Phe Glu Glu Arg Leu Ser Lys Lys Leu Phe Phe Phe Ser Gly
       50                  55                  60
   Arg Gln Val Trp Val Tyr Thr Gly Ala Ser Val Leu Gly Pro Arg Arg
   65                  70                  75                  80
   Leu Asp Lys Leu Gly Leu Gly Ala Asp Val Ala Gln Val Thr Gly Ala
                   85                  90                  95
   Leu Arg Ser Gly Arg Gly Lys Met Leu Leu Phe Ser Gly Arg Arg Leu
               100                 105                 110
   Trp Arg Phe Asp Val Lys Ala Gln Met Val Asp Pro Arg Ser Ala Ser
           115                 120                 125
   Glu Val Asp Arg Met Phe Pro Gly Val Pro Leu Asp Thr His Asp Val
       130                 135                 140
   Phe Gln Tyr Arg Glu Lys Ala Tyr Phe Cys Gln Asp Arg Phe Tyr Trp
   145                 150                 155                 160
   Arg Val Ser Ser Arg Ser Glu Leu Asn Gln Val Asp Gln Val Gly Tyr
                   165                 170                 175
   Val Thr Tyr Asp Ile Leu Gln Cys
               180